(19) **Europäisches Patentamt / European Patent Office / Office européen des brevets**

(11) **EP 4 759 935 A1**

(12) **EUROPEAN PATENT APPLICATION**
published in accordance with Art. 153(4) EPC

(43) Date of publication:
**17.06.2026 Bulletin 2026/25**

(21) Application number: **23949179.8**

(22) Date of filing: **16.08.2023**

(51) International Patent Classification (IPC):
*C12P 19/04* (2006.01)   *C12P 19/12* (2006.01)
*C12N 1/20* (2026.01)   *C12N 1/21* (2006.01)
*C12N 15/31* (2006.01)   *C12N 15/54* (2006.01)

(52) Cooperative Patent Classification (CPC):
C07K 14/195; C12N 1/20; C12N 9/10; C12N 15/74;
C12P 19/04; C12P 19/12

(86) International application number:
**PCT/JP2023/029646**

(87) International publication number:
**WO 2025/037411 (20.02.2025 Gazette 2025/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: Nippon Beet Sugar Manufacturing Co., Ltd.
**Tokyo 104-0031 (JP)**

(72) Inventors:
• **KIDO, Yusuke**
   **Obihiro-shi, Hokkaido 080-0831 (JP)**
• **NAGURA, Taizo**
   **Obihiro-shi, Hokkaido 080-0831 (JP)**
• **KONDO, Moemi**
   **Obihiro-shi, Hokkaido 080-0831 (JP)**

(74) Representative: **Prüfer & Partner mbB**
**Patentanwälte · Rechtsanwälte**
**Sohnckestraße 12**
**81479 München (DE)**

Remarks:
The complete document including Reference Table(s) and the Sequence Listing(s) can be downloaded from the EPO website

(54) **LINEAR INULIN SYNTHESIZING BACTERIUM AND METHOD FOR PRODUCING LINEAR INULIN**

(57)   [Problem] To provide: a linear inulin synthesizing bacterium of species closely related to the genus Bacillus, the bacterium being characterized in that a branched inulin synthase gene or a specific branched inulin synthase-like gene is deleted or inactivated and an inulosucrase gene or a specific inulosucrase-like gene is carried; a method for producing linear inulin using the linear inulin synthesizing bacterium; and others. [Solution] Only linear inulin can be produced by deleting or mutating the whole or a part of BI gene in an inulin synthesizing bacterium to produce a ΔBI inulin synthesizing strain in which BI enzyme has been deleted or inactivated, and then allowing a culture supernatant of the produced ΔBI inulin synthesizing strain to act on sucrose.

[Fig. 6]

## Description

[Technical Field]

The present invention relates to linear inulin synthesizing bacteria, and a method for producing a linear inulin, in greater detail, linear inulin synthesizing bacteria such as Priestia sp., Bacillus sp., Neobacillus sp., Fredinandcohnia sp., Metabacillus sp., Litchfieldia sp., Robertmurraya sp., Mycobacteroides sp., Alkalihalobacillus sp., Brevibacillus sp., Paenibacillus sp., and Ammoniphilus sp., as well as linear inulin synthesizing bacteria which are other than those linear inulin synthesizing bacteria and which are one, alternatively, two or more ones selected out of a group made up of linear inulin synthesizing bacteria being species closely related to Bacillus sp., which have a branching inulin synthesis-contributed enzyme gene or a given branching inulin synthesis-contributed enzyme-like gene deleted or inactivated, which have an inulosucrase gene or a given inulosucrase-like gene, as well as a matter like a method for producing a linear inulin by using at least either of those linear inulin synthesizing bacteria.

[Background Art]

Inulin is a kind of polysaccharide (fructan) present in roots of asteraceous plants such as chicory, has a sucrose structure at its terminus while being structured in a way where its fructose portion has other fructose residues polymerized, and has, for its feature, its bond between fructose residues being mainly β-2,1 bonds. In a broad sense, fructan having no glucose at its terminus and consisting of fructose only is also handled as inulin. Polymerization degrees of inulin in plants ranges approximately between 8 and 60. As for pathways in which inulin is synthesized in plant bodies, it is synthesized through action of two kinds of enzymes which are sucrose: sucrose 1-fructosyltransferase (1-SST) for carrying out fructosyl transfer between sucroses and 2,1-fructan: 2,1-fructan 1-fructosyltransferase (1-FFT) for carrying out such transfer between fructans whose polymerization degrees are 3 or more. Since a linear inulin whose bonds between fructose residues consist of β-2,1 bonds is hard to digest and to absorb and is utilized by Bifidobacteria in an alimentary canal to improve intestinal microflora and intestinal environments, it is blended as probiotics in health-orientated processed food. Additionally, since an inulin cream obtained by dissolving inulin in water to make it a creamy gel allows a texture like that of fats to be obtained, it is used for enhancing tastes of processed food which has fat decreased.

Chicory roots contain a linear inulin in abundance, and in producing it industrially, these are well used for raw material. In leading to the production, processes such as chicory cultivation, root harvesting, transport to a factory, root cleansing and shredding, hot water extraction, filtration, purification, concentrating, spray drying are caried out. For producing a linear inulin on an industrial scale, it is a first matter that a sufficient amount of raw material has to be secured while sufficient farmland area, long-term cultivation period from spring to autumn, sufficient fertilization management, and further, pest control such as chemicals appropriate for preventing disease damage are required. It is a second matter that since an inulin contained in harvested roots is easily degraded into low-weight molecules during storage and preservation while the roots themselves easily rot in storage, the roots, as raw material, need to be treated in a period as short as possible. This is why large-scale machinery equipment which allows a large amount of root to be treated in a short time is required for producing a linear inulin. Since the above reason makes production of a linear inulin require a great deal of labor and equipment, high cost of the production is problem and a linear inulin reasonable in price has been demanded by Industry field.

Meanwhile, it is reported that by using an enzyme called inulosucrase (hereinafter, referred to as 'IS' in some case) secreted by microorganisms, inulin is allowed to be synthesized from sucrose. Inulosucrase catalyzes a reaction of making β-2,1 transfer of a fructofuranosyl group of a sucrose to another sucrose or a terminus fructose portion of a product of that saccharide transfer, whereby inulin is synthesized from sucrose. As for examples of having synthesized inulin by using inulosucrase produced by means of genetic recombinant technology and inulosucrase having its purity enhanced by protein purification, those regarding lactic acid bacteria have been reported a lot. For example, Lactobacillus gasseri DSM20243 strain, L. gasseri DSM20604 strain, L. johnsonii NCC533 strain, L. reuteri 121 strain, L. reuteri TMW1.106 strain, Leuconostoc citreum CW28 strain, and Weissella confusa MBFCNC-2(1) strain have been reported on. Additionally, inulin syntheses by inulosucrase derived from Bacillus sp. 217C-11 strain and Salipaludibacillus agaradhaerens WDG185 strain, other than lactic acid bacteria, have been reported on (Non-patent Literature Document 1).

In a case of attempting to produce inulin industrially by using an enzyme of microorganisms, and in a case where the enzyme, which catalyzes a reaction, is required to be purified, cost increases to make it difficult to produce inulin at a low price. All inulosucrase producing bacteria which have been reported on so far secrete inulosucrase in a culture supernatant. In the present invention, microorganisms which secrete inulosucrase in a culture solution of the microorganisms and which generate inulin when this culture supernatant and sucrose are mixed with each other and are retained at a certain temperature for a given time or longer are referred to as inulin synthesizing bacteria. If a culture supernatant of inulin synthesizing bacteria can be used directly as an enzyme solution without being purified, inulin can be produced at a low price. As an example of having analyzed an inulin generated from sucrose by using a culture supernatant for an enzyme solution, Bacillus sp. 217C-11 has been reported on (Non-patent Literature Document 2). A generated product described on this report has been, due to an inulin peak pattern and shape on an HPLC based on an HPAEC-PAD method, thought of as a composition matter which also contains an inulin of a bonding manner different in structure from that of a linear inulin.

[Prior Art Documents]

[Non-patent Literature]

> [Non-patent Literature Document 1]
> Biotechnology Advances, 37, 306~318 (2019)
> [Non-patent Literature Document 2]
> J. Agric. Food Chem., 53, 1246~1253 (2005)

[Summary of Invention]

[Technical Problem]

In the present invention, an inulin which is generated in an enzymatic reaction using a culture supernatant (crude enzyme) of microorganisms with sucrose being a substrate, and which partially differs in structure is called a branching inulin. Although the branching inulin is a polymer which contains bonds other than $\beta$-2,1 ones for bonds between fructose residues in inulin, no microorganism has been yet reported on in nature world to give a culture supernatant which can generate a linear inulin exclusively.

That is, when a culture supernatant of inulin synthesizing bacteria is made to be an enzyme solution and react with sucrose, branching inulin is also generated inevitably and a linear inulin cannot be produced exclusively. Since to exclusively produce a linear inulin by using bacterial enzymes in accordance with conventional technology, it is essential to use inulosucrase protein partially or completely purified from a culture supernatant, which leads to significant elevation of production costs industrially. Additionally, causes for a branching inulin being contained in inulins generated when a culture supernatant is used as an enzyme solution have not yet been understood, and further, microorganisms which exclusively generate a linear inulin when culture supernatants of the microorganisms are used as enzyme solutions have not yet been found out. Meanwhile, it is technically facile to introduce, by using genetic recombinant technology, such an inulosucrase gene into non-inulin synthesizing bacteria to make them produce inulosucrase and to exclusively generate a linear inulin from sucrose. However, strict control is required as safety measures for environments to utilize industrially genetically recombinant microorganisms, which makes a new cost factor, and it requires discreet assurance of safety to use genetic recombinant technology in food field, and further, anxiety of consumers about food which has genetic recombinant technology used still remains deeply.

The present invention is intended to provide linear inulin synthesizing bacteria such as Priestia sp., Bacillus sp., Neobacillus sp., Fredinandcohnia sp., Metabacillus sp., Litchfieldia sp., Robertmurraya sp., Mycobacteroides sp., Alkalihalobacillus sp., Brevibacillus sp., Paenibacillus sp., and Ammoniphilus sp., as well as linear inulin synthesizing bacteria which are other than those linear inulin synthesizing bacteria and which are one, alternatively, two or more ones selected out of a group made up of linear inulin synthesizing bacteria being species closely related to Bacillus sp., which have a branching inulin synthesis-contributed enzyme gene or a given branching inulin synthesis-contributed enzyme-like gene deleted or inactivated, which have an inulosucrase gene or a given inulosucrase-like gene, as well as a matter like a method for producing a linear inulin by using at least either of those linear inulin synthesizing bacteria.

[Solution to Problem]

As a result of extensive research to solve the above problems, inventors of the present invention discovered, as a cause of an branching inulin being given out in a case of attempting to exclusively generate a linear inulin from sucrose by using a culture supernatant of inulin synthesizing bacteria, that a branching inulin synthesis-contributed enzyme (hereinafter, referred to as 'BI' in some case) which is an enzyme being unknown which transforms a linear inulin into a branching inulin is contained in addition to inulosucrase, which synthesizes a linear inulin from sucrose, and that by collaborative action of these two enzymes, the branching inulin is synthesized. On a basis of the discovery, they deleted or mutated all or some BI genes of inulin synthesizing bacteria and created linear inulin synthesizing bacteria (hereinafter, referred to as 'ΔBI inulin synthesizing bacterial strain' in some case) which have their BI enzymes deleted or inactivated, and found out that when a culture supernatant of the ΔBI inulin synthesizing bacterial strain created is made to act on sucrose, a linear inulin is exclusively generated, and accomplished each of the inventions below.

(1) Linear inulin synthesizing bacteria such as Priestia sp., Bacillus sp., Neobacillus sp., Fredinandcohnia sp., Metabacillus sp., Litchfieldia sp., Robertmurraya sp., Mycobacteroides sp., Alkalihalobacillus sp., Brevibacillus sp., Paenibacillus sp., and Ammoniphilus sp., as well as linear inulin synthesizing bacteria which are other than those linear inulin synthesizing bacteria and which are one, alternatively, two or more ones selected out of a group made up of linear inulin synthesizing bacteria being species closely related to Bacillus sp., which have a gene represented by at least any of (a)~(e) below deleted or inactivated, which have a gene represented by at least any of (h)~(m);

(a) a gene which encodes a protein made up of a base sequence having an identity of 70% or higher with a base sequence indicated by sequence number 35,
(b) a gene which hybridizes under stringent conditions with a complementary strand of a gene made up of a base sequence indicated by sequence number 35,
(c) a gene which encodes a protein made up of an amino acid sequence indicated by sequence number 36,
(d) a gene which encodes a protein made up of an amino acid sequence having 1~10 amino acid residues deleted, substituted, added, or inserted in an amino acid sequence indicated by sequence number 36,
(e) a gene which encodes a protein made up of an amino acid sequence having an identity of 67.9% or higher with an amino acid sequence indicated by sequence number 36,
(h) a gene which is of a base sequence indicated by sequence number 33,
(i) a gene which is made up of a base sequence having an identity of 70% or higher with a base sequence indicated by sequence number 33, as well as encodes a protein having an activity of catalyzing a reaction of making $\beta$-2,1 transfer of a fructofuranosyl group of a sucrose to another sucrose or a terminus fructose portion of a product of that saccharide transfer,
(j) a gene which hybridizes under stringent conditions with a complementary strand of a gene made up of a base sequence indicated by sequence number 33, as well as encodes a protein having an activity of catalyzing a reaction of making $\beta$-2,1 transfer of a fructofuranosyl group of a sucrose to another sucrose or a terminus fructose portion of a product of that saccharide transfer,
(k) a gene which encodes a protein that is made up of an amino acid sequence indicated by sequence number 34, and is to be microbe-extracellularly secreted,
(l) a gene which encodes a protein that is made up of an amino acid sequence having 1~10 amino acid residues deleted, substituted, added, or inserted in an amino acid sequence indicated by sequence number 34 as well as has an activity of catalyzing a reaction of making $\beta$-2,1 transfer of a fructofuranosyl group of a sucrose to another sucrose or a terminus fructose portion of a product of that saccharide transfer, and is to be microbe-extracellularly secreted, (m) a gene which encodes a protein that is made up of an amino acid sequence having an identity of 67.3% or higher with an amino acid sequence indicated by sequence number 34 as well as has an activity of catalyzing a reaction of making $\beta$-2,1 transfer of a fructofuranosyl group of a sucrose to another sucrose or a terminus fructose portion of a product of that saccharide transfer, and is to be microbe-extracellularly secreted.

(2) Linear inulin synthesizing bacteria according to (1), wherein linear inulin synthesizing bacteria of said inulin synthesizing bacteria have a feature shown in (f) or (g) below;

(f) Linear inulin synthesizing bacteria which generate an inulin which has its bonds between fructose residues be made up of $\beta$-2,1 bonds exclusively, by inoculating one platinum loop of microbial body into 3 mL of a culture medium whose composition is as below, shake-culturing it on conditions of 30°C, 24 hours, 130 rpm, and thereafter, making a culture supernatant thereof have sucrose added so as to be Bx40, and making it react at 37°C for 24 hours, <Culture medium composition>
10 g/L of sucrose, 5 g/L of soybean peptone, 1 g/L of yeast extract, 0.5 g/L of $KH_2PO_4$, adjusted to pH 7.2 with NaOH,
(g) Linear inulin synthesizing bacteria wherein when a reaction product obtained by inoculating one platinum loop of microbial body into 3 mL of a culture medium whose composition is as below (I), shake-culturing it on conditions of 30°C, 24 hours, 130 rpm, and thereafter, making a culture supernatant thereof have sucrose added so as to be Bx40, and making it react at 37°C for 24 hours is put in HPAEC-PAD analyses on conditions below (II), peaks of elution times identical to those of an inulin made up of $\beta$-2,1 bonds exclusively are seen at elution times of 20~30 minutes (polymerization degrees 9~16), while area of peaks other than said peaks are less than 1% of total peak areas,

<(I) Culture medium composition>
10 g/L of sucrose, 5 g/L of soybean peptone, 1 g/L of yeast extract, 0.5 g/L of $KH_2PO_4$, adjusted to pH 7.2 with NaOH,
<(II) Conditions of HPAEC-PAD analyses>
Column, CarboPac PA1 4 × 250 mm; Guard column, CarboPac PA1 4 × 50 mm; Injection amount, 5 μL of 0.1% water solution of a sample; Flow rate, 1.0 mL/minute; Elution, NaOH 100 mM constant, AcONa gradient (0~60 minutes, 0 → 600 mM); Detection, pulsed amperometry.

(3) Linear inulin synthesizing bacteria according to (1), wherein a gene made up of a base sequence which is of said sequence number 35 or which has an identity of 75% or higher with the sequence number has features shown below; When a protein made up of an amino acid sequence encoded by a gene made up of a base sequence which is of said sequence number 35 or which has an identity of 75% or higher with the sequence number, and a protein made up of an amino acid sequence indicated by sequence number 34 are respectively added to a Bx40 sucrose solution so as to be 2U/g sucrose and react at 37°C for 24 hours, and when a reaction product obtained is put in HPAEC-PAD analyses on conditions below, ten or more peaks of elution times identical to those of a linear inulin which is obtained by a reaction of a protein made up of an amino acid sequence indicated by sequence number 34 with a sucrose solution of Bx40, and which has its bonds between fructose residues be made up of β-2,1 bonds exclusively are obtained at elution times of 20 minutes or later (polymerization degree 9 or more), while ten or more peaks which appear between peaks of said linear inulin and are of a branching inulin having a structure where in a way of bonding other than β-2,1 bonds, a fructofuranosyl group is bonded to any fructose portion of a linear inulin are detected, and a branching inulin ratio (sum of peak area of branching inulin/ total peak area × 100) reaches 10% or higher at elution times of 20~30 minutes (polymerization degree 9~16),
<Conditions of HPAEC-PAD analyses>
Column, CarboPac PA1 4 × 250 mm; Guard column, CarboPac PA1 4 × 50 mm; Injection amount, 5 μL of 0.1% water solution of a sample; Flow rate, 1.0 mL/minute; Elution, NaOH 100 mM constant, AcONa gradient (0~60 minutes, 0 → 600 mM); Detection, pulsed amperometry.

(4) A purified enzyme, crudely-made enzyme, or enzyme-containing matter, culture solution, culture supernatant, or culture microbial body obtained from linear inulin synthesizing bacteria according to any of (1) to (3), or a treatment product thereof.

(5) A method for producing a linear inulin, which has a process of culturing linear inulin synthesizing bacteria according to any of (1) to (3).

(6) A method for producing a linear inulin, which has a process of obtaining a reaction product by making at least one of a purified enzyme, crudely-made enzyme, or enzyme-containing matter, culture solution, culture supernatant, or culture microbial body obtained from linear inulin synthesizing bacteria according to any of (1) to (3), or a treatment product thereof react with sucrose.

(7) A method for producing a linear inulin according to (6), wherein in a case of putting said reaction product in HPAEC-PAD analyses on conditions below, a ratio (sum of peak area of branching inulin/ total peak area × 100) of said branching inulin having a structure where in a way of bonding other than β-2,1 bonds, a fructofuranosyl group is bonded to any fructose portion of a linear inulin which has its bonds between fructose residues be made up of β-2,1 bonds is less than 1% at elution times of 20~30 minutes (polymerization degree 9~16),
<Conditions of HPAEC-PAD analyses>
Column, CarboPac PA1 4 × 250 mm; Guard column, CarboPac PA1 4 × 50 mm; Injection amount, 5 μL of 0.1% water solution of a sample; Flow rate, 1.0 mL/minute; Elution, NaOH 100 mM constant, AcONa gradient (0~60 minutes, 0 → 600 mM); Detection, pulsed amperometry.

(8) A method for producing a DFA III, which makes at least one of a purified enzyme, crudely-made enzyme, or enzyme-containing matter, culture solution, culture supernatant, or culture microbial body obtained from linear inulin synthesizing bacteria according to any of (1) to (3), or a treatment product thereof react with sucrose.

(9) A method for producing a DFA III according to (8), which uses inulin fructotransferase.

[Effect of Invention]

Linear inulin synthesizing bacteria according to the present invention allow, by their culture supernatant being used, a linear inulin exclusively to be synthesized from sucrose. Additionally, by using a highly pure linear inulin which is allowed by the present invention to be obtained and contains no branching inulin, a DFA III, which is a kind of oligosaccharide, is allowed to be produced in a high yield and at a reasonable price.

[Brief Description of Drawings]

[Fig.1] A diagram showing peaks based on HPAEC-PAD analyses for a reaction product obtained by making a culture supernatant of inulin synthesizing bacteria selected out of nature world react with Bx40 sucrose and for a plant (chicory)-derived inulin (Raftiline ST)

[Fig.2] A diagram showing a brief molecular phylogenetic tree based on 16s rDNA part base sequences for Bacillus sp. inulin synthesizing bacteria identified and closely related to Bacillus drentensis

[Fig.3] A diagram showing peaks based on HPAEC-PAD analyses for reaction products obtained by making two kinds of sucrose metabolism related enzymes purified out of a culture supernatant of an SD33 strain react with Bx40 sucrose and for a plant-derived inulin (Raftiline ST)

[Fig.4] A diagram showing $^{13}$C-nuclear magnetic resonance (NMR) signals of a levan preparation, an inulin preparation, a reaction product at Peak A, and a reaction product at Peak B

[Fig.5] A diagram showing GC-MS peaks of respective methylation analyses for a reaction product at Peak A, a reaction product at Peak B, and Peak 2 for the reaction product at Peak B [Fig.6] A diagram showing peaks based on HPAEC-PAD analyses for reaction products obtained by making IS and BI of an SD33 strain resulting from recombinant production of E. coli react with Bx40 sucrose

[Fig.7] A molecular phylogenetic tree created by using 16S rDNA part base sequences of Ammoniphilus sp. YIM 78166 strain, Bacillus subtilis type strain and an SD33 strain, which are type strains of microorganisms obvious in species name, out of microorganism groups having base sequences high in homology with those of IS and BI of the SD33 strain

[Fig.8] A diagram of peaks based on respective HPAEC-PAD analyses for reaction products obtained by making respective culture supernatants of an SD33 strain and an SD33_ΔBI strain react with Bx40 sucrose

[Fig.9] A diagram of peaks based on respective HPAEC-PAD analyses for reaction products obtained by mixing a culture supernatant of an SD33_ΔBI strain with various purified BI-like recombinant enzymes to make them react with Bx40 sucrose

[Fig.10] A diagram of peaks based on respective HPAEC-PAD analyses for reaction products obtained by making a culture supernatant of an SD33_ΔBI strain, as well as making OV191_IS (67.3% in homology with SD33 strain IS) and LL01_IS (68.8% in homology with SD33 strain IS), react with Bx40 sucrose

[Description of Embodiments]

Below, linear inulin synthesizing bacteria according to the present invention, a purified enzyme, crudely-made enzyme, or enzyme-containing matter, culture solution, culture supernatant, or culture microbial body obtained from linear inulin synthesizing bacteria according to the present invention, or a treatment product thereof, a method for manufacturing a linear inulin, and a method for manufacturing a DFA III shall be explained in detail. Linear inulin synthesizing bacteria according to the present invention are linear inulin synthesizing bacteria such as Priestia sp., Bacillus sp., Neobacillus sp., Fredinandcohnia sp., Metabacillus sp., Litchfieldia sp., Robertmurraya sp., Mycobacteroides sp., Alkalihalobacillus sp., Brevibacillus sp., Paenibacillus sp., and Ammoniphilus sp., as well as linear inulin synthesizing bacteria which are other than those linear inulin synthesizing bacteria and which are one, alternatively, two or more ones selected out of a group made up of linear inulin synthesizing bacteria being species closely related to Bacillus sp., which have a branching inulin synthesis-contributed enzyme (BI) gene or a given branching inulin synthesis-contributed enzyme-like (BI-like) gene deleted or inactivated, which have an inulosucrase (IS) gene or a given inulosucrase-like (IS-like) gene.

Although inventors of the present invention separated and collected numerous inulin synthesizing bacteria from nature world, used culture supernatants of those to keenly search for bacteria exclusively generating a linear inulin from sucrose, and scrutinized inulin products whose substrate is sucrose with culture supernatants of numerous inulin synthesizing bacteria being objects, all the inulin synthesizing bacteria generated a branching inulin, and inulin synthesizing bacteria generating a linear inulin exclusively were not found out.

Then, as a result of further keen research, it has been tracked down that as a cause of a branching inulin is given out when a culture supernatant of inulin synthesizing bacteria is used, an unknown protein exist. Specifically, it has been discovered that in a culture supernatant of inulin synthesizing bacteria, an enzyme being unknown which transforms a linear inulin into a branching inulin is contained in addition to IS, which synthesizes a linear inulin from sucrose. This enzyme being unknown is BI. Further, it has been tracked down that by collaborative action of these two enzymes, IS and BI, the branching inulin is synthesized.

Then, although by a Basic Local Alignment Search Tool (BLAST) search being carried out with Non-redundant protein sequence (nr) being a database, a bacterial species which retains a base sequence encoding an IS-like protein while not retaining a base sequence encoding a BI-like protein newly found out has been searched for, such a bacterial species has not yet been found out, and it has been found out that all bacteria which has base sequences encoding amino acid sequences by 67.3% or higher in homology with an amino acid sequence of IS, indicated by sequence number 34, retains base sequences encoding amino acid sequences by 66.9% or higher in homology with an amino acid sequence of BI, indicated by sequence number 36, and further that even a protein which is by 67.9% in homology with BI gives out a branching inulin when it is, together with IS, made to react with sucrose. As bacterial species which retain both base sequences encoding an IS-like protein and a BI-like protein as above, and can generate a branching inulin, Priestia sp., Bacillus sp., Neobacillus sp., Fredinandcohnia sp., Metabacillus sp., Litchfieldia sp., Robertmurraya sp., Mycobacteroides sp., Alkalihalobacillus sp., Brevibacillus sp., Paenibacillus sp., and Ammoniphilus sp., that is, species closely related to Bacillus sp. have been specified.

Next, by deleting or mutating all or some BI genes of inulin synthesizing bacteria, linear inulin synthesizing bacteria (hereinafter, ΔBI inulin synthesizing bacterial strain) which have their BI enzymes deleted or inactivated were created, and when a culture supernatant of the ΔBI inulin synthesizing bacterial strain created was made to act on sucrose, success has been made in generating a linear inulin exclusively, and the present invention has been completed. As bacterial species suitable for creating a ΔBI inulin synthesizing bacterial strain, Priestia sp., Bacillus sp., Neobacillus sp., Fredinandcohnia sp., Metabacillus sp., Litchfieldia sp., Robertmurraya sp., Mycobacteroides sp., Alkalihalobacillus sp., Brevibacillus sp., Paenibacillus sp., and Ammoniphilus sp., and so on, stated in advance, can be used. In other words, Priestia sp., Bacillus sp., Neobacillus sp., Fredinandcohnia sp., Metabacillus sp., Litchfieldia sp., Robertmurraya sp., Mycobacteroides sp., Alkalihalobacillus sp., Brevibacillus sp., Paenibacillus sp., and Ammoniphilus sp., that is, species closely related to Bacillus sp. can be mentioned, and additionally, it can be said that with regard to those bacterial species, production of a linear inulin where culture supernatants thereof are used cannot be accomplished, without breeding improvement being carried out.

In the present invention, 'IS-like gene' refers to a gene which corresponds to sequence number 33 and encodes a protein having an activity of an inulosucrase catalyzing a reaction of making $\beta$-2,1 transfer of a fructofuranosyl group of a sucrose to another sucrose or a terminus fructose portion of a product of that saccharide transfer. Specifically, a gene made up of a base sequence having an identity of at least 70% or higher, preferably 75% or higher, more preferably 80% or higher, further more preferably 85% or higher, specially preferably 90% or higher, and most preferably 95% or higher with a base sequence of sequence number 33 is mentioned. Further, a gene which is made up of a base sequence where in the above gene, one or several base sequences are deleted, substituted, added, or inserted and which has a functionality identical to that of the above gene will also do.

Additionally, a gene which hybridizes under stringent conditions with a complementary strand of a gene made up of a base sequence indicated by sequence number 33 as well as encodes a protein having an activity of catalyzing a reaction of making $\beta$-2,1 transfer of a fructofuranosyl group of a sucrose to another sucrose or a terminus fructose portion of a product of that saccharide transfer. The 'stringent condition' in the present description means a condition where a complementary strand of a gene strand having homology with a target sequence preferentially hybridizes with the target sequence while a complementary strand of a gene strand having no homology therewith does not substantially hybridize therewith. Specifically, a matter like carrying out hybridization for one night under conditions of 65°C, doing a wash for eliminating nonspecific reactions for 5 minutes under conditions of room temperature by using 2×SSC, and thereafter, doing twice a 30-minute wash repeatedly under conditions of 65°C by using 0.2×SSC containing 0.1%SDS is mentioned as a stringent condition. The 2×SSC is, for its composition, 0.3 mol/L of NaCl and 30 mmol/L of sodium citrate (pH7.0).

Linear inulin synthesizing bacteria which have their activity of BI-like protein deleted or inactivated can be obtained by deleting or inactivating a BI-like gene on a base sequence of a parent strain. Here, in the present invention, the 'BI-like gene' means a DNA made up of a transcription region including an ORF and a transcription regulatory region such as a promoter of the gene, and specifically, refers to a gene which corresponds to sequence number 35 and encodes a protein of an enzyme which catalyzes a reaction of transferring a fructofuranosyl group of a sucrose to any fructose portion of a linear inulin in a way of bonding other than $\beta$-2,1 bonds and synthesizing a branching inulin. More specifically, a gene which encodes a protein made up of an amino acid sequence having an identity of at least 70% or higher, preferably 75% or higher, more preferably 80% or higher, further more preferably 85% or higher, specially preferably 90% or higher, and most preferably 95% or higher with a base sequence of sequence number 35 is mentioned. Further, a gene which is made up of a base sequence where in the above gene, one to several base sequences are deleted, substituted, added, or inserted and which has a functionality identical to that of the above gene will also do. The 'one to several' can be, for example, 1~10, preferably 1~7, and more preferably 1~4. In the present invention, the 'linear inulin' means a fructan which has a structure where to a fructose portion of a sucrose, other fructose residues are polymerized in $\beta$-2,1 bonds and which has its bonds between fructose residues be made up of $\beta$-2,1 bonds exclusively. The 'bonding other than $\beta$-2,1 bonds' is literally a 'bonding other than $\beta$-2,1 bonds' and means a bonding other than $\beta$-2,1 bonds, at least such as $\beta$-2,3 bonds, $\beta$-2,4 bonds, besides $\beta$-2,6 bonds.

Additionally, a gene which, under stringent conditions, hybridizes with a gene made up of a base sequence indicated by sequence number 35 while encodes a protein of an enzyme which catalyzes a reaction of transferring a fructofuranosyl group of a sucrose to any fructose portion of a linear inulin in a way of bonding other than $\beta$-2,1 bonds and synthesizing a branching inulin is included in BI-like genes, in the present invention.

As for 'deleting or inactivating a BI-like gene or a BI gene,' to lower an activity of a BI-like protein or BI protein transcribed/translated, and to preclude a BI-like protein or BI protein from being produced, by introducing mutation into one or more bases on a base sequence of the gene, that is, by a matter like partially or wholly deleting a base sequence of the gene, or substituting or inserting, with respect to the base sequence, another base sequence, as well as to make microbe-extracellular secretion production capability of a BI-like protein or BI protein be defective by introducing mutation into a base sequence which is usually present on a 5' terminus side of a BI-like gene or a BI gene and encodes a signal peptide are mentioned in the present invention.

Next, although an inulosucrase catalyzing a reaction of making β-2,1 transfer of a fructofuranosyl group of a sucrose to another sucrose or a terminus fructose portion of a product of that saccharide transfer in the present invention is made up of an amino acid sequence indicated by sequence number 34, a protein of an amino acid sequence having an identity of 67.3% or higher with the above amino acid sequence can be used as far as it has that activity. Specifically, a protein made up of an amino acid sequence having an identity of at least 67.3% or higher, preferably 70% or higher, more preferably 75% or higher, further more preferably 80% or higher, much further more preferably 85% or higher, specially preferably 90% or higher, most preferably 95% or higher, and more specifically, a protein made up of an amino acid sequence having an identity of at least 67.3% or higher, and preferably 68.0% or higher, 68.6% or higher, 68.8% or higher, 70.3% or higher, 70.9% or higher, 71.7% or higher, 73.4% or higher, 76.8% or higher, 78.7% or higher, 78.9% or higher, 79.1% or higher, 79.4% or higher, 79.8% or higher, 80.4% or higher, 80.7% or higher, 81.1% or higher, 81.3% or higher, 81.7% or higher, 81.9% or higher, 83.4% or higher, 84.9% or higher, 85.8% or higher, 96.2% or higher, 96.8% or higher, 97.3% or higher, 98.2% or higher, 98.4% or higher, 98.6% or higher are mentioned.

Although an enzyme which catalyzes a reaction of transferring a fructofuranosyl group of a sucrose to any fructose portion of a linear inulin in a way of bonding other than β-2,1 bonds and synthesizing a branching inulin in the present invention is made up of an amino acid sequence indicated by sequence number 36, a protein of an amino acid sequence having an identity of 67.9% or higher with the above amino acid sequence can be used as far as it has that activity. Specifically, a protein made up of an amino acid sequence having an identity of at least 67.9% or higher, preferably 70% or higher, more preferably 75% or higher, further more preferably 80% or higher, much further more preferably 85% or higher, specially preferably 90% or higher, most preferably 95% or higher, and more specifically, a protein made up of an amino acid sequence having an identity of at least 67.9% or higher, and preferably 71.5% or higher, 72.2% or higher, 75.1% or higher, 75.3% or higher, 76.6% or higher, 76.9% or higher, 77.1% or higher, 77.3% or higher, 77.5% or higher, 78.3% or higher, 78.5% or higher, 78.7% or higher, 79.0% or higher, 80.8% or higher, 81.0% or higher, 84.8% or higher, 85.3% or higher, 85.4% or higher, 85.8% or higher, 86.0% or higher, 88.6% or higher, 89.7% or higher, 93.1% or higher, 93.7% or higher, 96.6% or higher, 98.7% or higher, 98.9% or higher, 99.1% or higher are mentioned.

Furthermore, one which is made up of an amino acid sequence where in an amino acid sequence indicated by sequence number 34 and sequence number 36, one to several amino acids are deleted, substituted, added, or inserted and which has a functionality identical to that of the above protein will also do. Here, the 'one to several' can be, for example, 1~10, preferably 1~7, and more preferably 1~4.

Here, for 'activity of a BI-like protein or BI protein being deleted or inactivated,' it will do to eliminate BI-like genes or BI genes themselves from on genomes, and it will do to introduce a mutation causing inactivation or lowered activity of BI by a matter like substituting, deleting, and inserting bases. Additionally, it will also do to make microbe-extracellular secretion production capability of BI be defective by eliminating a base sequence which is usually present on a 5' terminus side of a BI-like gene or a BI gene and encodes a signal peptide.

As procedures of 'deleting or inactivating a BI-like protein or BI protein', a method of obtaining linear inulin synthesizing bacteria whose BI activity has vanished or been lowered in an appropriate method after giving a random mutation of genomes is mentioned besides a method of deleting or inactivating a BI-like gene or a BI gene, with a plan.

It suffices to use, for example, a method by means of homologous recombination, for a method for planned deletion or inactivation of a BI-like gene or a BI gene. That is, by making a circular recombinant plasmid obtained by inserting into an appropriate plasmid vector, a DNA fragment containing sequences upstream and downstream of a BI-like gene or a BI gene be taken into a parent strain cell, and through two-time crossover homologous recombination in an outer region of the BI-like gene or the BI gene, a BI-like gene on a genome of a parent strain can be deleted. Alternatively, by building up in a method such as PCR, a linear DNA fragment where inactivating mutation by means of base substitution, base insertion, and so on is introduced into a BI-like gene or a BI gene, and making it be taken into a parent strain cell and making it cause a two-time crossover homologous recombination in regions of two outer locations of mutation locations in a BI-like gene or a BI gene of a parent strain genome, a BI-like gene or a BI gene on a genome is enabled to be replaced by an inactivated gene fragment. Additionally, it will also do to carry out deletion, substitution, and base insertion on a BI-like gene or a BI gene by means of genome editing technology and so on.

Meanwhile, as a method for deleting or inactivating genes randomly, a method of treating a parent strain with mutagenic agents such as nitrosoguanidine (NTG) and ethyl methanesulfonate (EMS) and a method of irradiating it with UV, heavy-ion beam, and so on are mentioned, for example.

By culturing linear inulin synthesizing bacteria having a BI-like gene or a BI gene deleted or inactivated in a method described above, a culture solution having a BI-like protein or BI protein deleted or inactivated can be obtained.

In a case where a reaction product is obtained by a culture supernatant of the culture solution being made to react with sucrose, a linear inulin accounts for a majority in the reaction product. In a case where inulin is obtained from the reaction product and is put in HPAEC-PAD analyses on conditions below, ten or more is detected for peaks of a linear inulin at elution times of 20 minutes or later, and if it is supposed that a branching inulin is given out similarly as in a case of paragraph 0043 later described where inulin is generated from sucrose by a culture supernatant of a parent strain being used, peaks thereof appear between peaks of a linear inulin, and at elution times of 20~30 minutes (polymerization degree 9~16), a branching inulin ratio (sum of peak area of branching inulin/ total peak area $\times$ 100) is lower than 10% and is preferably 7% or lower, more preferably 5% or lower, further more preferably 3% or lower, specially preferably 1% or lower, and most preferably 0.3% or lower. Here, in the present invention, 'reaction product' refers to what is generated by a substrate being transformed by an activity which IS or IS and BI has, and a reaction product generated by a substrate being transformed by an activity which IS has includes, besides a reaction product containing a linear inulin, a linear inulin just as it is, and a reaction product generated by a substrate being transformed by an activity which IS and BI has includes, besides a reaction product containing a linear inulin and a branching inulin, a linear inulin and a branching inulin just as they are. Since a technique of obtaining a linear inulin or a branching inulin contained in a reaction product is not limited in particular, a person skilled in the art can adopt a technique which he or she can select as appropriate.

<Conditions of HPAEC-PAD analyses>

Column, CarboPac PA1 4 $\times$ 250 mm; Guard column, CarboPac PA1 4 $\times$ 50 mm; Injection amount, 5 $\mu$L of 0.1% water solution of a sample; Flow rate, 1.0 mL/minute; Elution, NaOH 100 mM constant, AcONa gradient (0~60 minutes, 0 $\rightarrow$ 600 mM); Detection, pulsed amperometry.

A parent strain used for obtaining linear inulin synthesizing bacteria according to the present invention is not limited in particular when it is inulin synthesizing bacteria having a gene of base sequences recognized as being by at least 60% or higher identical to those of sequence number 33 and sequence number 35, respectively. Specifically, a bacillus such as Priestia sp., Bacillus sp., Neobacillus sp., Fredinandcohnia sp., Metabacillus sp., Litchfieldia sp., Robertmurraya sp., Mycobacteroides sp., Alkalihalobacillus sp., Brevibacillus sp., Paenibacillus sp., and Ammoniphilus sp. and species closely related to Bacillus sp. other than those can be used. More specifically, Priestia megaterium, Bacillus sp., Neobacillus bataviensis, Metabacillus bambusae, Robertmurraya korlensis, Fredinandcohnia onubensis, Litchfieldia salsa, Mycobacteroides abscessus subsp. abscessus, Brevibacillus sp., Brevibacillus formosus, Brevibacillus brevis, Brevibacillus dissolubilis, Brevibacillus migulae, Paenibacillus sp., Paenibacillus elgii, Paenibacillus prosopidis, Paenibacillus tyrfis, Ammoniphilus sp., Alkalihalobacillus krulwichiae, and Salipaludibacillus agaradhaerens can be mentioned, and further more specifically, Bacillus sp. SD33 strain (accession number: NITE BP-03890) can be mentioned. According to molecular phylogenetic tree analyses based on 16S rDNA part base sequences, type strains of microorganism species above mentioned which are other than Mycobacteroides abscessus subsp. abscessus, as well as Ammoniphilus sp. YIM 78166 strain are all mapped at positions near those of Bacillus subtilis (most common Bacillus sp. bacteria). Since Brevibacillus sp. and Paenibacillus sp. are customarily handled as species closely related to Bacillus sp., it is reasonable that at least, microorganism species above mentioned which are other than Mycobacteroides abscessus subsp. abscessus are handled as species closely related to Bacillus sp.. Bacillus sp. SD33 strain is, on May 22, 2023, deposited to NITE Patent Microorganisms Depositary, National Institute of Technology and Evaluation (2-5-8 Kazusakamatari, Kisarazu city, Chiba prefecture) and granted an accession number: NITE BP-03890.

In a case where in a case of having made a culture supernatant of the parent strain react with sucrose to have obtained a reaction product and obtained inulin from the reaction product, the inulin obtained is put to HPAEC-PAD analyses on conditions below, ten or more are, at elution times of 20 minutes or later, detected for peaks of a branching inulin and peaks of a linear inulin respectively, with peaks of the branching inulin appearing between peaks of a linear inulin and a branching inulin ratio (sum of peak area of branching inulin/ total peak area $\times$ 100) reaches 10% or higher at elution times of 20~30 minutes (polymerization degree 9~16).

<Conditions of HPAEC-PAD analyses>

Column, CarboPac PA1 4 $\times$ 250 mm; Guard column, CarboPac PA1 4 $\times$ 50 mm; Injection amount, 5 $\mu$L of 0.1% water solution of a sample; Flow rate, 1.0 mL/minute; Elution, NaOH 100 mM constant, AcONa gradient (0~60 minutes, 0 $\rightarrow$ 600 mM); Detection, pulsed amperometry.

Although a culture medium suitable for culturing linear inulin synthesizing bacteria according to the present invention contains a carbon source, a nitrogen source, an inorganic salt, and so on, it is not one limited by these, and besides, nutrition sources used for an ordinary culture such as amino acids and vitamins can be also used as appropriate, out of necessity. As carbon sources to be added to a culture medium, carbon sources publicly known in this field, for example, saccharides such as sucrose, glucose, fructose, and maltose can be used. However, in a case of using Bacillus sp. SD33 strain for a microorganism, it is most preferable to use a liquid culture medium making sucrose be its main carbon source, which can enhance a production amount of an inulin synthesizing enzyme according to the present invention. These carbon sources can be used alone or as mixed, at adequate concentrations (e.g. 5~50 g/L). Additionally, these carbon sources may be both in a mode of being isolated/ purified and in a mode of being contained in another matter, and for example, it is allowable to use a sucrose-containing matter such as sucrose crude sugar or waste molasses instead of sucrose purification product in a case of making sucrose be a carbon source. Although as nitrogen sources to be added to a culture medium, for example, organic nitrogen sources such as peptone, meat extract, yeast extract, corn steep liquor, and besides, inorganic nitrogen sources such as ammonium salts of sulfuric acid, nitric acid, and phosphoric acid can be used alone or as mixed, limitation to these is not present. Additionally, although as inorganic salts, for example, sulfate salts, hydrochloride salts, carbonate salts, nitrate salts, phosphate salts, and so on of potassium, sodium, calcium, magnesium, manganese, iron, and so on can be respectively used alone or as combined, limitation to these is not present.

As for pH, a culture medium having the above composition is pH6~9, preferably pH7~8. Additionally, a culture of said microorganism can be carried out in a shake culture or in an aeration and agitation culture. A culturing temperature is preferably 25~35°C. Additionally, a culturing time is when being a time or longer in which microorganisms can proliferate, good and is 5~96 hours, preferably, 15~72 hours.

Next, although linear inulin synthesizing bacteria according to the present invention microbe-extracellularly secrete a protein that is made up of an amino acid sequence indicated by sequence number 34, and is to be microbe-extracellularly secreted, a protein that is made up of an amino acid sequence having 1~10 amino acid residues deleted, substituted, added, or inserted in an amino acid sequence indicated by sequence number 34 as well as has an activity of catalyzing a reaction of making $\beta$-2,1 transfer of a fructofuranosyl group of a sucrose to another sucrose or a terminus fructose portion of a product of that saccharide transfer, and is to be microbe-extracellularly secreted, and/or a protein that is made up of an amino acid sequence having an identity of 67.3% or higher with an amino acid sequence indicated by sequence number 34 as well as has an activity of catalyzing a reaction of making $\beta$-2,1 transfer of a fructofuranosyl group of a sucrose to another sucrose or a terminus fructose portion of a product of that saccharide transfer, and is to be microbe-extracellularly secreted, that is, IS and/or IS-like, a manner thereof may be a protein, that is, an enzyme, and even an enzyme-containing matter. As for a manner in a case of being an enzyme, a purified enzyme and a crudely-made enzyme can be mentioned, and in a case of being an enzyme-containing matter, an enzyme-containing matter, or a treatment product of the enzyme-containing matter, and besides, a culture solution, culture supernatant, or culture microbial body, or a treatment product thereof are also allowable. Additionally, any of those purified enzyme, crudely-made enzyme, or enzyme-containing matter, culture solution, culture supernatant, or culture microbial body, or a treatment product thereof is a substance obtained from linear inulin synthesizing bacteria according to the present invention, and as for an obtaining method thereof, an obtaining method which can be selected as appropriate by a person skilled in the art can be adopted, including microbe-extracellular secretion.

Additionally, a method for producing inulin, according to the present invention has a process of (i) and /or (ii) below.

(i) a process of culturing linear inulin synthesizing bacteria according to the present invention,
(ii) a process of making at least one of a purified enzyme, crudely-made enzyme, or enzyme-containing matter, culture solution, culture supernatant, or culture microbial body obtained from linear inulin synthesizing bacteria according to the present invention, or a treatment product thereof react with sucrose.

Since a process (i) above, that is, 'a process of culturing linear inulin synthesizing bacteria according to the present invention' is not limited in particular as long as the linear inulin synthesizing bacteria according to the present invention can be cultured, a culturing method which can be selected as appropriate by a person skilled in the art can be adopted for culturing linear inulin synthesizing bacteria according to the present invention, and as such a culturing method, for example, a method of inoculating linear inulin synthesizing bacteria according to the present invention into a liquid culture medium making sucrose be its main carbon source, and culturing them under conditions of pH 7~8 and 25~35°C for 15~72 hours can be mentioned.

A process (ii) above, that is, 'a process of making at least one of a purified enzyme, crudely-made enzyme, or enzyme-containing matter, culture solution, culture supernatant, or culture microbial body obtained from linear inulin synthesizing bacteria according to the present invention, or a treatment product thereof react with sucrose' is a process of making a purified enzyme, crudely-made enzyme, or enzyme-containing matter, culture solution, culture supernatant, or culture microbial body obtained in an obtaining method stated above, or a treatment product thereof react with sucrose. Since a technique of said 'reaction' is not limited in particular as long as it allows a linear inulin to be produced, a technique of reaction, which can be selected as appropriate by a person skilled in the art can be adopted, and as such a technique of 'reaction', a method of adding at least one of a purified enzyme, crudely-made enzyme, or enzyme-containing matter, culture solution, culture supernatant, or culture microbial body obtained from linear inulin synthesizing bacteria according to the present invention, or a treatment product thereof to a reaction solution containing sucrose, and making it react can be mentioned, for example.

As for a process (ii) above, that is, a method for producing a linear inulin, having 'a process of obtaining a reaction product by making at least one of a purified enzyme, crudely-made enzyme, or enzyme-containing matter, culture solution, culture supernatant, or culture microbial body obtained from linear inulin synthesizing bacteria according to the present invention, or a treatment product thereof react with sucrose' stated above, HPAEC-PAD analyses of (iii) below can be carried out for the reaction product obtained, further.

(iii) wherein in a case of putting said reaction product (obtained in a process (ii) above) in HPAEC-PAD analyses on conditions below, a ratio (sum of peak area of branching inulin/ total peak area $\times$ 100) of said branching inulin having a structure where in a way of bonding other than $\beta$-2,1 bonds, a fructofuranosyl group is bonded to any fructose portion of a linear inulin which has its bonds between fructose residues be made up of $\beta$-2,1 bonds is less than 1% at elution times of 20~30 minutes (polymerization degree 9~16);

<Conditions of HPAEC-PAD analyses>

Column, CarboPac PA1 4 $\times$ 250 mm; Guard column, CarboPac PA1 4 $\times$ 50 mm; Injection amount, 5 $\mu$L of 0.1% water solution of a sample; Flow rate, 1.0 mL/minute; Elution, NaOH 100 mM constant, AcONa gradient (0~60 minutes, 0 $\rightarrow$ 600 mM); Detection, pulsed amperometry.

Next, linear inulin synthesizing bacteria according to the present invention allow, in a production of substances whose raw materials are inulin or fructan, such as difructose dianhydride III (DFA III), inulooligosaccharides, and cycloinulooligosaccharides (cyclofructan), those to be directly produced from sucrose, by being used in parallel with a culture supernatant thereof. Especially for producing DFA III, inulin fructotransferase (hereinafter, referred to as 'IFT') can be used, and besides, at least one of a purified enzyme, crudely-made enzyme, or enzyme-containing matter, culture solution, culture supernatant, or culture microbial body obtained from microorganisms producing IFT, or a treatment product thereof (hereinafter, referred to as 'IFT and so on') can be used. Additionally, DFA III can be produced by combining at least one of a purified enzyme, crudely-made enzyme, or enzyme-containing matter, culture solution, culture supernatant, or culture microbial body obtained from linear inulin synthesizing bacteria according to the present invention, or a treatment product thereof with IFT and so on, and making them react with sucrose, and by selecting such a method for producing DFA III, DFA III are allowed to be directly produced from sucrose. In a production of DFA III stated above, DFA III can be produced at high yield in a case where IFT and so on is made to act on a linear inulin as compared with a case where IFT and so on is made to act on an inulin containing a branching inulin.

Additionally, as microorganisms generating IFT, microorganisms belonging to Arthrobacter sp., Kluyveromyces sp., Streptomyces sp., Enterobacter sp., Bacillus sp., and Microbacterium sp. and besides, various species of bacteria, yeasts, mycelia, actinomycetes, and so on can be used as apprppriate, and for example, Arthrobacter ureafaciens, same (IFO12140), same (ATCC21124), A. pascens (IFO12139), same T13-2, A. globiformis (IFO12137), same C11-1, A. nictinovorans GS-9, A. ilicis OKU 17B, Arthrobacter sp., same H65-7, same AHU1753 (FERM BP-8296), same MCI-2493; Kluyveromyces marxianus (ATCC12424), same CBS6556, K. marxianus var. marxianus, same (IFO1735); Streptomyces fumigatus, S. rochei, same E87, Streptomyces sp., same MCI-2524; Pseudomonas fluorescens, same No.949; Bacillus circulans, same OKUMZ. 31b, same MCI-2554, Bacillus sp., same Snu-7; Aureobacterium sp., same MCI-2494; Microbacterium sp., same AL-210; Enterobacter sp., same S45; Aspergillus fumigatus; Penicillium purpurogenum can be mentioned.

Below, linear inulin synthesizing bacteria, a purified enzyme, crudely-made enzyme, or enzyme-containing matter, culture solution, culture supernatant, or culture microbial body obtained from linear inulin synthesizing bacteria according to the present invention, or a treatment product thereof, a method for producing a linear inulin, and a method for producing DFA III is described on a basis of embodiment examples. Technical scope of the present invention is not limited to embodiments shown by these embodiment examples.

[Embodiment Example]

Embodiment Example 1: Searching for linear inulin synthesizing bacteria in nature world In the present Embodiment Example 1, microbes which synthesize a linear inulin exclusively were searched for in nature world. Below, procedures and results are described in detail.

Soil samples collected from various locations of Japan were suspended with sterile water, smeared onto basal agar culture media, and cultured for 24 hours under conditions of 35°C. Colonies which came out on agar culture media were inoculated into 300 $\mu$L of a basal culture medium, and were statically cultured for 48 hours under conditions of 35°C. 40 $\mu$L of culture supernatants were added to 160 $\mu$L of a 10 mM sodium phosphate buffer solution (pH 7.0) containing 10% sucrose, and were made to react for 24 hours under conditions of 35°C. That was made to be sample 1. 2 $\mu$L of an Aspergillus niger derived exoinulinase (made by Sigma-Aldrich) was added to 40 $\mu$L of the sample 1, and was retained for 24 hours under conditions of 35°C. That was made to be sample 2. The samples 1 and 2 were put in TLC analyses, and colony strains where a polysaccharide spot at an origin point, recognized in the sample 1, vanished in the sample 2 were made to be inulin synthesizing bacteria, and 100 of the strains were obtained in total. Conditions of TLC analyses were made to be 'Plate: TLC Silica Gel 60 (Merck), Load amount: 50 $\mu$g, Mobile phase: 2-propanol : 1-butanol : $H_2O$ = 2 : 2 : 1,

Development: 10 cm, Color development: Spraying reagents (p-anisaldehyde : acetic acid : concentrated sulfuric acid : ethanol = 13 : 5: 18 : 478), and thereafter, heating for 3 minutes under conditions of 105°C'.

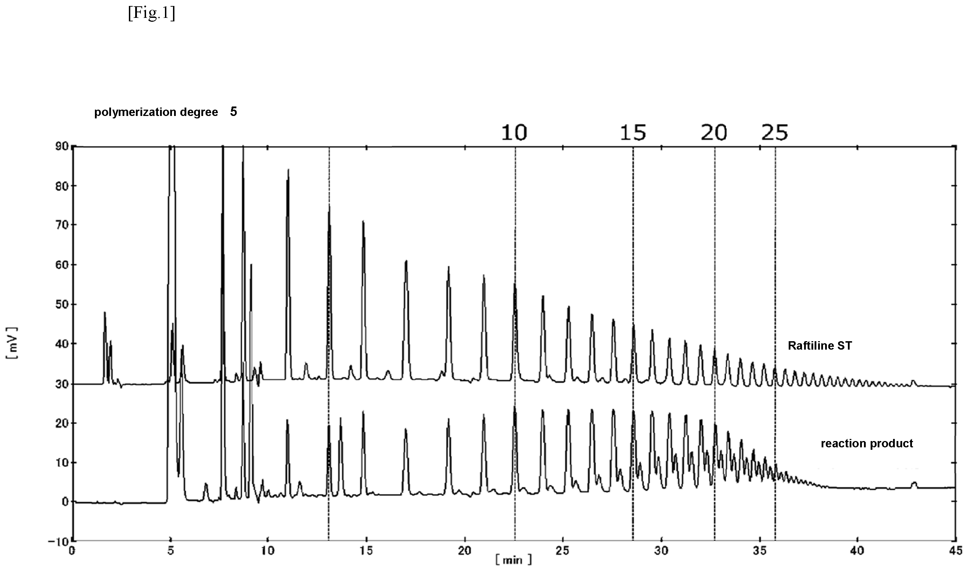

Out of the 100 strains obtained, 20 strains which rank upper in amount of enzyme secretion into a culture supernatant were selected. Next, these 20 strains of inulin synthesizing bacteria were inoculated into 3 mL of a basal culture medium, and were shake-cultured on conditions of 30°C, 24 hours, 130 rpm. The culture supernatant was collected by means of a centrifugation of 12000 × g, 5 minutes. After the culture supernatant was measured in enzyme activity, it was mixed with a Bx40 sucrose solution so as to be 2U/g sucrose, and was retained for 24 hours under conditions of 37°C. This reaction solution was retained for 5 minutes under conditions of 100°C to have its enzyme deactivated, and was put in HPAEC-PAD analyses. Peaks based on HPAEC-PAD analyses respective for a reaction product obtained and for a plant-derived inulin (Raftiline ST) are shown in Fig.1. The enzyme activity was calculated on a basis of a generation amount of reducing sugar measured in a DNS method. That is, after 100 $\mu$L of the reaction solution was made to be in composition of an enzyme solution (10 $\mu$L) diluted as appropriate, 300 mM of sucrose, and 40 mM of a MES-NaOH buffer solution (pH 6.5), and to react for 10 minutes under conditions of 37°C, it had 100 $\mu$L of a DNS reagent (16 g/L of NaOH, 5 g/L of 3,5-dinitrosalycylic acid, 300 g/L of potassium sodium tartrate) added, was heated for 20 minutes under conditions of 105°C, and was made to develop color, and thereafter, was measured in absorbance at 535 nm. A reducing sugar amount in a reaction solution is obtained from a standard curve made by using 0~5 mM of D-glucose solution, and an enzyme amount at which 1 $\mu$mol of reducing sugar is generated under the present conditions has been defined as 1U. HPAEC-PAD analyses in embodiment examples below were carried out by using an HPAEC-PAD device (ICS-3000), made by Dionex, on conditions of Column, CarboPac PA1 4 × 250 mm; Guard column, CarboPac PA1 4 × 50 mm; Injection amount, 5 $\mu$L of 0.1% water solution of a sample; Flow rate, 1.0 mL/minute; Elution, NaOH 100 mM constant, AcONa gradient (0~60 minutes, 0 $\rightarrow$ 600 mM).

As shown in Fig.1, a pattern of fructan peaks of polymerization degree 9 or higher (HPLC elution times of minutes between 20 and 30), out of a peak pattern of a reaction solution, was different, and in all reaction solutions, resulted in a peak pattern of a polysaccharide, which cannot be seen for a plant-derived inulin (Raftiline ST).

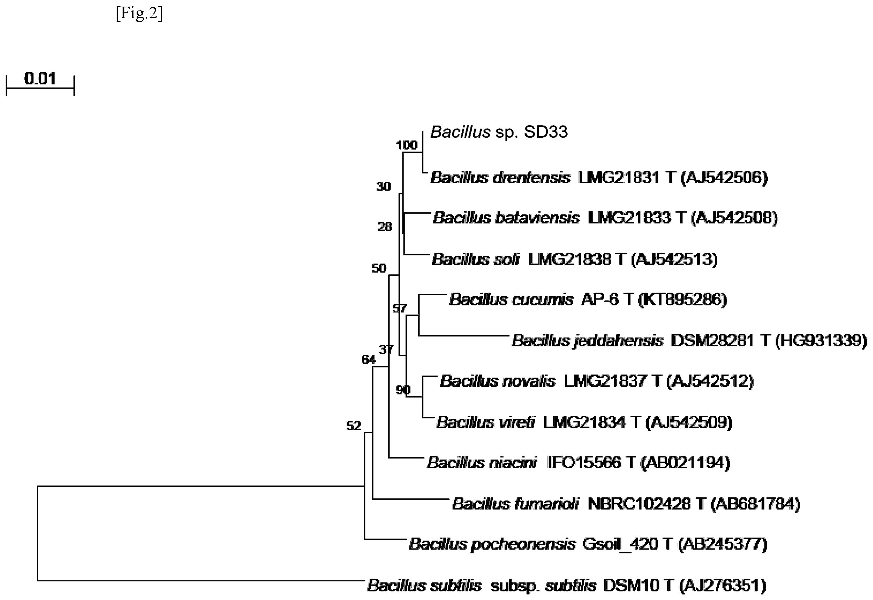

Additionally, one strain of inulin synthesizing bacteria obtained has been identified as Bacillus sp. bacteria closely related to Bacillus drentensis, on a basis of a DNA base sequence analysis/ molecular phylogenetic analysis (16S rDNA-full, entrusted to TechnoSuruga Laboratory Co., Ltd.). Bacillus drentensis has been reclassified into Neobacillus drentensis since 2020 (Int. j. Syst. Evol. Microbiol., 70, 406~438 (2020)). A brief molecular phylogenetic tree based on 16S rDNA part base sequences for Bacillus sp. inulin synthesizing bacteria identified and closely related to Bacillus drentensis is shown in Fig.2. As shown in Fig.2, this strain is made to be Bacillus sp. SD33 strain (hereinafter, SD33 strain) (accession number: NITE BP-03890).

Embodiment Example 2: Specifying a cause gene which generates a branching inulin

In the present Embodiment Example 2, a cause why inulin synthesizing bacteria found out in Embodiment Example 1 generate a branching inulin and cannot generate a linear inulin exclusively has been clarified.

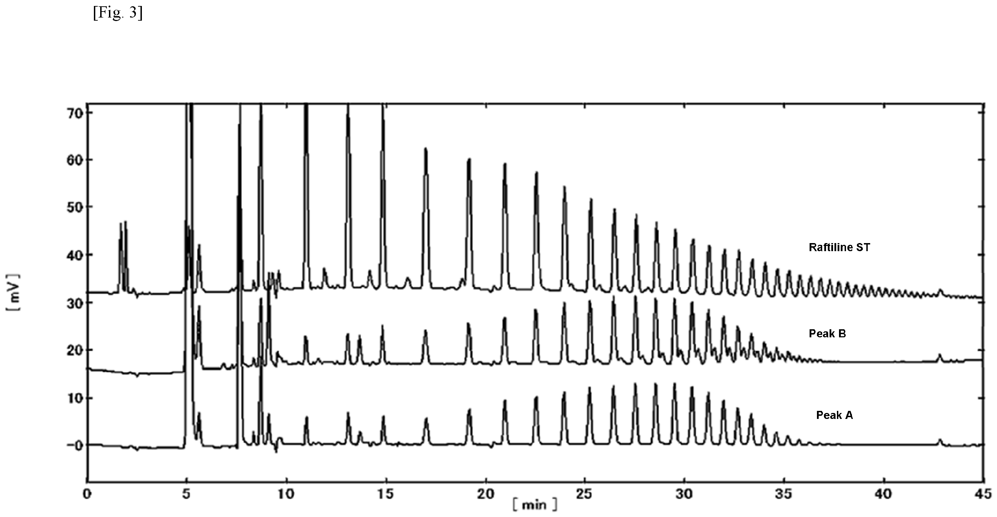

SD33 strains were smeared onto basal agar culture media, and were cultured for one night under conditions of 35°C. A single colony obtained was inoculated into 6 mL of a basal culture medium, and was precultured on conditions of 30°C, 24 hours, 130 rpm. 2.5 ml of this was each inoculated into 500 mL × 2 of the same culture medium stuck onto a 2L capacity conical flask, cultured on condition of 30°C, 24 hours, 130 rpm, and thereafter, a culture supernatant was collected by means of a centrifugation of 12000 × g, 20 minutes. The culture supernatant obtained had ammonium sulfate added so as to be 70% saturated, and was retained for one night under conditions of 4°C while being agitated. After an ammonium sulfate precipitation product of the culture supernatant was collected by means of a centrifugation of 12000 × g, 20 minutes, was dissolved in a 10 mM sodium phosphate buffer solution (pH 7.0), and was put in a dialysis tube, it was dialyzed sufficiently with the same buffer solution, and a crude enzyme solution was obtained. The crude enzyme solution was loaded onto Toyopearl DEAE 650M (2.5 cm i.d. × 20 cm, Tosoh Corporation) equilibrated in advance with a 10 mM sodium phosphate buffer solution (pH 7.0), and a non-adsorption fraction was eluted with the same buffer solution. Because the non-adsorption fraction was seen to have an activity of freeing reducing sugar from sucrose, the non-adsorption fraction had ammonium sulfate added so as to be 80 mM. and was loaded onto Toyopearl butyl 650M (2.5 cm i.d. × 10 cm, Tosoh Corporation) equilibrated in advance with a 10 mM sodium phosphate buffer solution (pH 7.0) containing 80 mM ammonium sulfate, and after the non-adsorption fraction was eluted with the same buffer solution, adsorption fraction was eluted in a linear gradient of ammonium sulfate concentration 80 mM $\rightarrow$ 0 mM. In the present operation, two activity peaks A, B indicating an activity of freeing reducing sugar from sucrose were obtained. For a fraction solution of each of the peaks obtained, a reaction solution was, with regard to a sample where each of them had been added to Bx40 sucrose so as to be 2U/g sucrose, prepared in a method described in Embodiment Example 1, and a product having its enzyme deactivated was analyzed by using HPAEC-PAD. Respective peaks of a reaction product at Peak A, a reaction product at Peak B, and a plant-derived inulin (Raftiline ST) are shown in Fig.3.

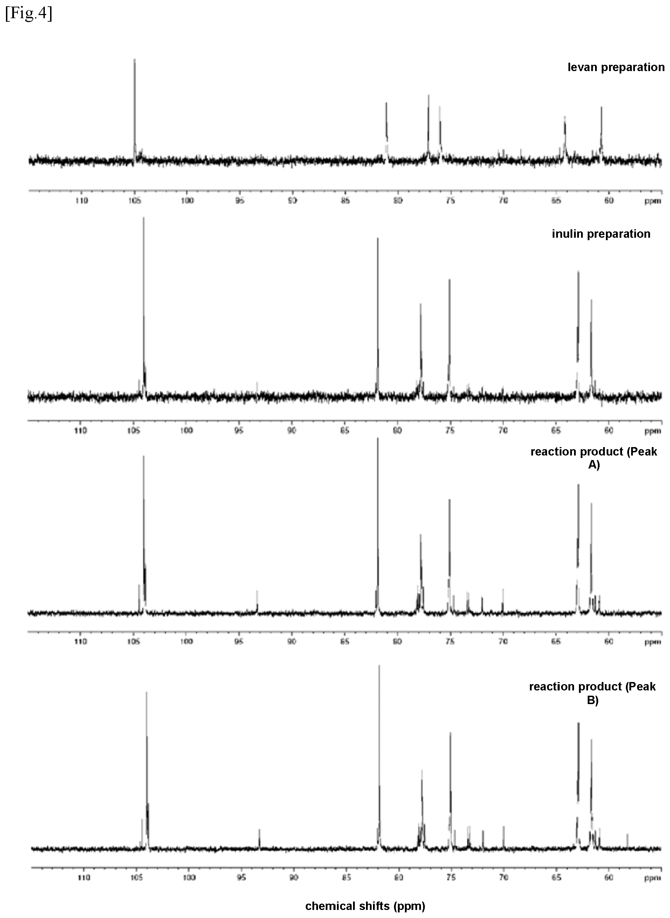

As shown in Fig.3, in characteristics of a peak pattern, a reaction product at Peak A accorded with a linear inulin, and a reaction product at Peak B did with an inulin containing a branching inulin. Next, a structure of each of the products was analyzed with nuclear magnetic resonance (NMR). Firstly, 1 ml of each reaction solution had 99.5% of ethanol added so as to be 50% in final concentration, and after polysaccharides were precipitated, precipitate was collected by means of a centrifugation of 12000 × g, 2 minutes. After the precipitate collected had 2 mL of 50% ethanol added again to be suspended, precipitate was collected by means of a centrifugation of 12000 × g, 2 minutes. By doing five times the present operation repeatedly, low molecular compounds were eliminated. The precipitate obtained was lyophilized and had $^{13}$C-NMR carried out with inulin (chicory-derived, Raftiline HP) and levan (produced by Zymomonas mobilis, Sigma-Aldrich) being a standard preparation. Respective $^{13}$C-NMR signals of a levan preparation, an inulin preparation, a reaction product at Peak A, and a reaction product at Peak B are shown in Fig.4.

As shown in Fig.4, all the products represented a $^{13}$C-NMR signal almost identical with that of an inulin preparation. From this, it was thought that reaction products at Peak A and Peak B had both a basic structure identical with that of inulin, and that partial differences in structure made a difference in inulin between being linear and being branching. Since protein at Peak A is contained for all the peaks, it has been tracked down that protein at Peak A is IS, and that protein contained for Peak B exclusively is a causal enzyme BI which generates a branching inulin.

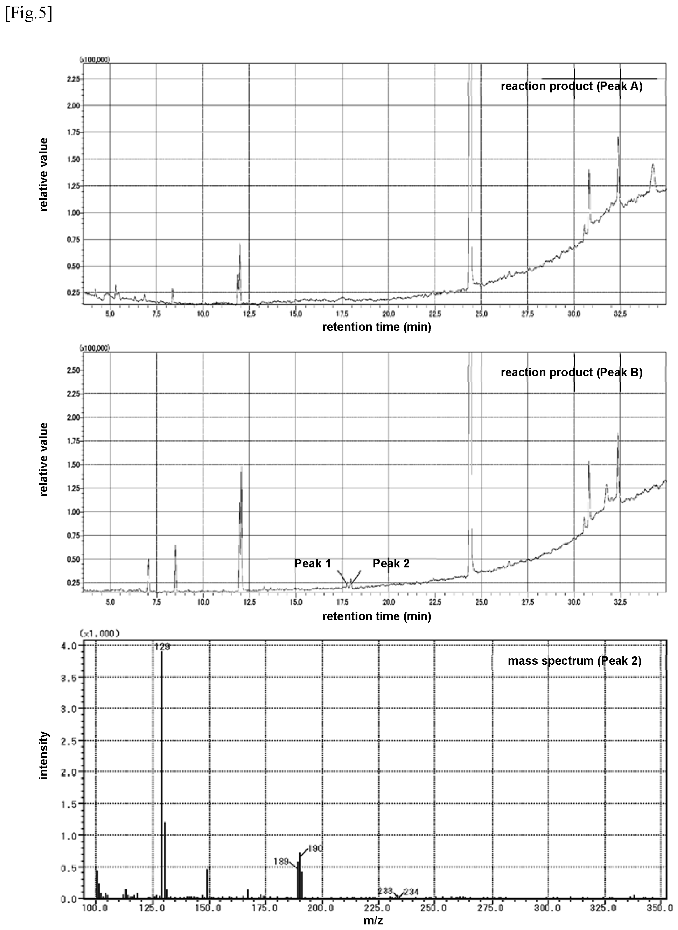

Next, in a method described in 'Nature Protocols, 7, 1590~1607 (2012)', free hydroxyl groups of 50 mg of a reaction product at Peak A and of a reaction product at Peak B were all methylated, methylated polysaccharides were hydrolyzed, and thereafter, partially methylated saccharides freed were reduced with NaBD$_4$ and were acetylated, and partially methylated alditol acetates (PMAAs) were obtained. The PMAAs obtained were put in GC-MS analyses. The GC-MS analyses were carried out by using a GC-MS spectrometer (GCMS-QP2010 Ultra), made by Shimadzu Corporation, with a capillary column, BPX70 (25 m × 0.22 mm inner diameter, film = 0.25 $\mu$m; made by SGE Analytical Science); Injection amount, 1 $\mu$L (split ratio 1/10); Vaporization chamber temperature, 240°C; Carrier gas, helium; Flow rate, 1.0 mL/minute; Column temperature, multistep (0~2 minutes, 170°C; 2~32 minutes, 170→260°C, 32~35 minutes, 260°C), for GC, whereas on conditions of Ion source temperature, 230°C; Interface temperature, 250°C; Solvent elution time, 3 minutes; Measurement mode, scan; m/z, 100~350, for MS. GC-MS peaks of respective methylation analyses for a reaction product at Peak A, a reaction product at Peak B, and Peak 2 for the reaction product at Peak B are shown in Fig.5.

As shown in Fig.5, a few GC-MS peaks which were not included for a reaction product at Peak A were seen for a reaction product for Peak B. Out of those, Peak 2 was thought of as being for 1,2,5,6-tetra-O-acetyl-(2-deuterio)-3,4-di-O-methyl glucitol since m/z 189 and 190 were for main primary fragments, m/z 233 and 234 for minor fragments, and m/z 129 for a sole secondary fragment. That is, it was thought that in a branching inulin, at least a branching structure of β-2,6 bonds is contained.

Next, in order to obtain gene sequence information on IS and BI, N-terminus amino acid sequences of proteins included for Peaks A and B were determined in accordance with operation below. Firstly, after SDS-polyacrylamide gel electrophoresis (10%) for each of the peaks was carried out in accordance with an ordinary technique, transcription was made on a PVDF film and CBB dying was carried out. Next, after films for target band parts (any of them 44 kDa) were cut out and cleansed in an order of MeOH, 50% MeOH, and ultrapure water, the N-terminus amino acid sequence was analyzed by means of Procise 492 (Applied Biosystems). As a result, it was presumed that Peak A was of IS while Peak B was of a mixture of IS and BI. An N-terminus amino acid sequence of IS was determined to be Ala-Glu-Ile-Ser-Ser-Asp-Tyr-Thr-Ser-Ile-Trp, and an N-terminus amino acid sequence of BI was presumed to be Glu-Asp-Val-Gly-Gln-Thr-Thr-Asn-Trp.

Next, base sequences of an IS gene and a vicinity thereof was in accordance with an ordinary technique. That is, a matter at Peak A was degraded by lysyl endopeptidase, and peptides obtained were separated by means of a reverse-phase HPLC, and had their inner partial sequences analyzed. On a basis of the inner partial sequences, a few sense- and antisense primers were made, and a PCR was carried out with a genome DNA of an SD33 strain being a template. A PCR in order to amplify DNA fragments was, by using a PrimeSTAR Max DNA Polymerase, carried out by means of a DNA amplification using an iCycle Thermal Cycler (Bio-Rad Laboratories). A PCR reaction solution was prepared by adding 1 $\mu$L of template DNA diluted as appropriate, 20 pmol respectively of sense- and antisense primers, and 25 $\mu$L of PrimeSTAR Max Premix (2×) and further, making a total amount of reaction solution be 50 $\mu$L. Conditions for PCR reactions were made to be one cycle of 30 seconds under conditions of 98°C, and 30 cycles of three-step temperature changes of 10 seconds under conditions of 98°C, 5 seconds under conditions of 55°C, and 5~30 seconds (adjusted in accordance with target amplification products, guidelines of 5 seconds per 1 kb) under conditions of 72°C.

A primer of an outward direction was made on a basis of a sequence of an amplification fragment obtained, and base sequences upstream and downstream were determined by means of an adaptor PCR using an LA PCR in vitro Cloning Kit (Takara Bio). Here, it is predetermined that 'upstream' and 'downstream' of a gene do not refers to a position with respect to a replication origin, the upstream refers to a region continuous to a 5' side of an initiation codon of a gene being a subject in each manipulation/ process, whereas the downstream refers to a region continuous to a 3' side of a termination codon of a gene being a subject in each manipulation/ process, and hereinafter, the same holds.

Results are shown in sequence number 37. When an open reading frame (ORF) was searched for in vicinity sequences of the IS gene obtained, it has been made evident that an ORF (hereinafter, ORF1) presumed to be BI is present immediately downstream of IS. Additionally, it has been made evident that any has a signal peptide sequence on its N-terminus side.

Next, E. coli recombinant enzyme expression systems were built up on a basis of base sequences encoding mature proteins of an IS gene and an ORF1, which had their signal peptides removed. That is, PCRs were carried out by making SD33 strain genome DNAs be templates and using primer pairs for extensions of IS and ORF1 sequences, which are shown in Table 1 below, and a base sequence of each of the mature proteins whose N-terminus sides had initiation codons, and whose C-terminus sides had a His-tag for affinity purification, added respectively, were amplified.

[Table 1]

| Primer name | Sequence (5'-3') | Sequence number |
|---|---|---|
| SD33 IS s | GGAGATATACATATGTTTGCAGCGGAAATCAGT | 1 |
| SD33 IS as | GTGGTGGTGGTGGTGGCGATTTGCCCCAAATGG | 2 |
| SD33 ORF1 s | GGAGATATACATATGGAAGACGTAGGGCAAACG | 3 |
| SD33 ORF1 as | GTGGTGGTGGTGGTGTTGTTTAATCCAGCCAGG | 4 |

A PCR in order to amplify DNA fragments was in accordance with statements of the present Embodiment Example 2. Amplification fragments obtained were bonded to an Nde I - His-tag region of Plasmid pET-22b (Merck Millipore) by using Takara In-fusion HD (Takara Bio). The plasmid was made to be produced by E. coli DH5α transformants and was purified in an alkali SDS method. After sequence correctness of the plasmid was assured by means of a sequence analysis using a T7 promoter and a T7 terminator, which are universal primers, E. coli BL21 (λDE3) was transformed by using the plasmid obtained and was cultured on an LB agar culture medium containing 200 μg/mL of ampicillin, for 18 hours under conditions of 37°C. After colonies obtained were inoculated into 3 mL of an LB liquid culture medium containing 200 μg/mL of ampicillin and was cultured for one night on conditions of 37°C, 130 rpm, a culture solution thereof was inoculated into 50 mL of the same liquid culture medium in an amount of 1/100 and was cultured on conditions of 37°C, 160 rpm until OD600 = 0.5. This was statically placed on ice for 10 minutes, had 50 μL of 0.1 M of IPTG added (final concentration 0.1 mM), and was cultured for 18 hours on conditions of 18°C, 120 rpm. Successively, after microbial bodies were collected by means of a centrifugation of 5000 × g, 10 minutes, they were suspended in a 10 mM sodium phosphate buffer solution (pH 7.5) and had ultrasonic disruption (duty cycle 50, output 5, 2 minutes × 3) carried out by using Sonifier 450 (Branson). After they were again given a centrifugation of 12000 × g, 10 minutes, a supernatant was loaded onto an Ni-chelating sepharose column (φ1.0 × 4 cm) equilibrated in advance with a 10 mM sodium phosphate buffer solution (pH 7.5) containing 50 mM imidazole. After a non-adsorption fraction was cleansed with the same buffer solution, imidazole concentration was heightened to 250 mM and adsorbed proteins were eluted, and these were made to be a IS and an ORF1 purified enzyme.

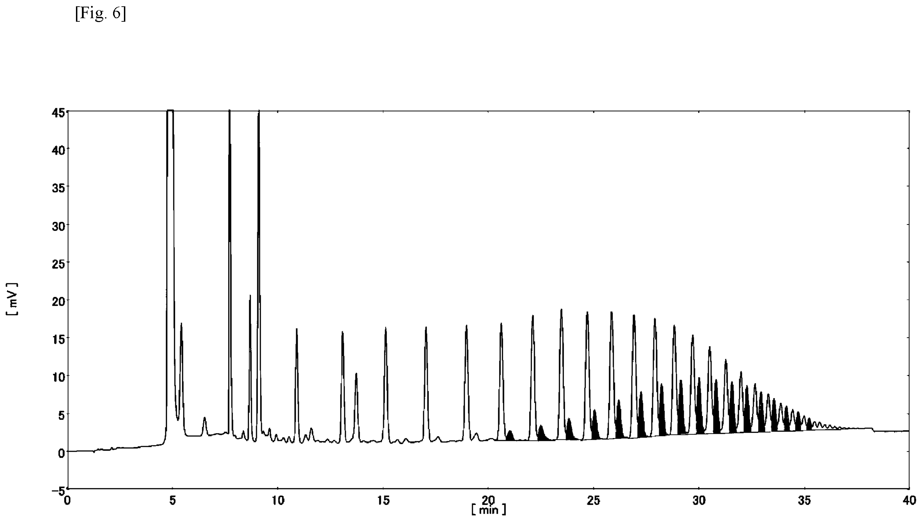

When an IS purified enzyme was made to react with Bx40 sucrose so as to be 2U/g sucrose, a linear inulin was generated. Meanwhile, it was analyzed, in a method described in Embodiment Example 1, that when IS and an ORF1 purified enzyme were mixed so as to be 2U/g sucrose respectively and were made to react with Bx40 sucrose, a branching inulin was generated. Peaks based on HPAEC-PAD analyses for reaction products obtained by making IS and BI of an SD33 strain react with Bx40 sucrose are shown in Fig.6.

As shown in Fig.6, it was made certain that a count of branching inulin peaks detected between linear inulin peaks at elution times of 20 minutes or later under the present analysis conditions were 10 or more, and that a branching inulin ratio (sum of peak area of branching inulin/ total peak area × 100) at elution times of 20~30 minutes (polymerization degree 9~16) was 23.9% and higher than 10% (Fig 6). When the ORF1 purified enzyme was alone made to react with Bx40 sucrose, products of saccharide transfer, which were 3~5 in polymerization degree, were seen. From the above, an ORF1 has been specified as being a gene which encodes BI protein. Homology between an amino acid sequence (sequence number 34) of IS and an amino acid sequence (sequence number 36) of BI was 40.2%.

Embodiment Example 3: Searching for inulin synthesizing bacteria holding no BI-like gene, by means of BLAST Search

In the present Embodiment Example 3, inulin synthesizing bacteria which do not hold a gene encoding a causal protein (BI) made evident in Embodiment Example 2 was searched for by means of BLAST Search, and it has been made evident that 'bacteria holding a gene encoding a protein having a homology over a given degree with IS all hold a gene encoding a protein having a homology over a given degree with a causal protein (BI)'.

In order to search for inulin synthesizing bacteria holding no BI-like gene, a BLAST search was carried out for amino acid sequences of IS and BI of an SD33 strain, which are indicated by sequence number 34 and sequence number 36, by using Non-redundant protein sequences (nr) as a data base. Results thereof are shown in Table 2 below. In Table 2, microorganism strains (protein resulting from recombinant production) having a base sequence encoding a protein by 67.3% or higher in homology with IS are represented in bold characters.

[Table 2]

| Scientific name | Homology (BI) | Homology (IS) | GenBank Accession No. (BI) | GenBank Accession No. (IS) |
|---|---|---|---|---|
| *Bacillus* sp. AFS031507 | 99.1 | 98.6 | WP_098930308.1 | WP_098930566.1 |
| *Priestia megaterium* | 98.9 | 98.4 | WP_168242085.1 | WP_168242084.1 |
| *Bacillus* sp. MM2020_1 | 98.7 | 98.2 | WP_166256440.1 | WP_166256437.1 |
| *Bacillus* sp. ISL-46 | 96.6 | 97.3 | WP_251026627.1 | WP_214731329.1 |
| *Bacillus* sp. X1(2014) | 93.7 | 96.2 | WP_144554311.1 | WP_144554313.1 |
| *Neobacillus Bataviensis* | 93.1 | 96.8 | WP_223589392.1 | WP_223589390.1 |
| *Fredinandcohnia onubensis* | 89.7 | 84.9 | **WP_099354679.1 (F. onubensis_BI)** | WP_099354678.1 |
| *Metabacillus bambusae* | 88.6 | 85.8 | WP_207980430.1 | WP_243459527.1 |
| *Alkalihalobacillus krulwichiae* | 86.0 | 68.6 | **WP_066151830.1 (A. krulwichiae_BI)** | WP_066151831.1 |
| *Litchfieldia salsa* | 85.8 | 83.4 | WP_090856254.1 | WP_090856257.1 |
| *Mycobacteroides abscessus* subsp. *abscessus* | 85.4 | 81.3 | **SHR63422.1 (M. abscessus_BI)** | SHR63461.1 |
| *Bacillus* sp. Y1 | 85.3 | 81.7 | WP_119706533.1 | WP_119706532.1 |
| *Robertmurraya Korlensis* | 84.8 | 80.4 | **WP_066052166.1 (R. korlensis_BI)** | WP_066052162.1 |
| *Brevibacillus formosus* | 81.0 | 81.1 | KLH99468.1 | KLH99469.1 |
| *Brevibacillus formosus* | 80.8 | 80.7 | MBG9945611.1 | MBG9945612.1 |
| *Brevibacillus* sp. RS1.1 | 79.0 | 79.4 | WP_173627228.1 | WP_173627227.1 |
| *Brevibacillus brevis* | 79.0 | 79.1 | **WP_064202059.1 (B. brevis_BI)** | WP_064202058.1 |
| *Ammoniphilus* sp. YIM 78166 | 78.7 | 79.8 | WP_134704859.1 | WP_134704861.1 |
| *Brevibacillus* sp. HB2.2 | 78.7 | 79.4 | WP_173604213.1 | WP_173604212.1 |
| *Brevibacillus formosus* | 78.7 | 79.4 | **WP_088909523.1 (B. formosus_BI)** | WP_088909524.1 |
| *Brevibacillus* sp. M2.1A | 78.7 | 78.7 | WP_173611151.1 | WP_173611150.1 |
| *Brevibacillus dissolubilis* | 78.5 | 81.9 | WP_139492467.1 | WP_139492529.1 |
| *Brevibacillus formosus* | 78.5 | 79.4 | WP_083995063.1 | WP_052773388.1 |
| *Brevibacillus* | 78.3 | 78.9 | WP_106780220.1 | WP_106780218.1 |

(continued)

| Scientific name | Homology (BI) | Homology (IS) | GenBank Accession No. (BI) | GenBank Accession No. (IS) |
|---|---|---|---|---|
| *Brevibacillus migulae* | 77.5 | 76.8 | WP_134686959.1 | WP_134686958.1 |
| *Paenibacillus* sp. A3 | 77.3 | 73.4 | WP_054972566.1 | WP_054972565.1 |
| *Paenibacillus* sp. WST5 | 77.3 | 71.7 | WP_188172561.1 | WP_188172560.1 |
| *Paenibacillus elgii* | 77.1 | 73.4 | **WP_108533520.1 (P. elgii_BI)** | WP_108533519.1 |
| *Paenibacillus tyrfis* | 76.9 | 73.4 | GLI10887.1 | GLI10888.1 |
| *Paenibacillus elgii* | 76.6 | 73.4 | WP_163855095.1 | WP_163855093.1 |
| *Paenibacillus prosopidis* | 75.3 | 68.0 | WP_245975758.1 | WP_245975758.1 |
| *Paenibacillus* sp. OV191 | 75.1 | 67.3 | **PYE68991.1 (OV191_BI)** | **PYE68991.1 (OV191_IS)** |
| *Paenibacillus* sp. YIM B00362 | 72.2 | 70.9 | WP_199616132.1 | WP_199616134.1 |
| *Paenibacillus* sp. A3 | 71.5 | 70.3 | WP_171649235.1 | WP_171649234.1 |
| *Bacillus* sp. LL01 | 67.9 | 68.8 | **WP_047973383.1 (LL01_BI)** | **WP_053073957.1 (LL01_IS)** |
| *Salipaludibacillus agaradhaerens* WDG185 | 66.9 | 67.4 | **ATN45517.1 (S. agaradhaerens_BI)*** | ATN45518.1 |
| *Normal recombinant production was not possible in E. coli. | | | | |

Firstly, as a result of specifying a bacterial strain having a base sequence (IS-like gene; sequence number 33) encoding a protein by 67.3% or higher (specifically, 67.3%, 68.0%, 68.6%, 68.8%, 70.3%, 70.9%, 71.7%, 73.4%, 76.8%, 78.7%, 78.9%, 79.1%, 79.4%, 79.8%, 80.4%, 80.7%, 81.1%, 81.3%, 81.7%, 81.9%, 83.4%, 84.9%, 85.8%, 96.2%, 96.8%, 97.3%, 98.2%, 98.4%, 98.6%) homologous to an amino acid sequence of IS, it has been made evident that all the bacterial strains have a base sequence (BI-like gene; sequence number 35) encoding a protein by 67.9% or higher (specifically, 67.9%, 71.5%, 72.2%, 75.1%, 75.3%, 76.6%, 76.9%, 77.1%, 77.3%, 77.5%, 78.3%, 78.5%, 78.7%, 79.0%, 80.8%, 81.0%, 84.8%, 85.3%, 85.4%, 85.8%, 86.0%, 88.6%, 89.7%, 93.1%, 93.7%, 96.6%, 98.7%, 98.9%, 99.1%) homologous to an amino acid sequence of BI. Additionally, bacterial strains listed in Table 2 above all accorded in that they have, like SD33 strains, a BI-like gene placed immediately downstream of an IS-like gene. Since Paenibacillus prosopidis and Paenibacillus sp. OV191 strain had no termination codon seen in an IS-like gene and had no signal peptide-like sequence seen on an N-terminus side of a BI-like gene, a possibility of BI-like protein and IS-like protein being produced in concatenation with each other was considered. As above, it has been made evident that bacteria holding IS-like genes having homology over a given degree all hold BI-like genes. Homology in amino acid sequence between IS and each BI-like protein and that therein between BI and each IS-like protein were 50% and lower, respectively.

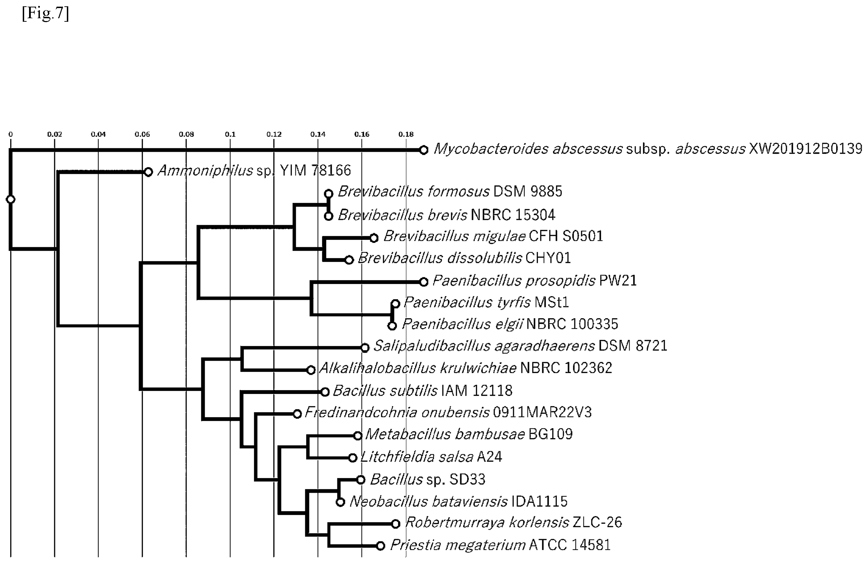

A molecular phylogenetic tree was made by putting 16S rDNA part base sequences of Ammoniphilus sp. YIM 78166 strain, Bacillus subtilis type strain and SD33 strain, which are type strains of microorganisms obvious in species name, out of microorganisms listed in Table 2 above in multiple comparison by means of ClustalW, and utilizing FastTree v2.1.8, and using a default parameter. The molecular phylogenetic tree made is shown in Fig.7.

As shown in Fig.7, a type strain of each microorganism species other than Mycobacteroides abscessus subsp. abscessus, as well as Ammoniphilus sp. YIM78166 strain are all mapped at a position near Bacillus subtilis (most general Bacillus sp. bacteria). Since Brevibacillus sp. and Paenibacillus sp. are customarily handled as species closely related to Bacillus sp., it has been shown that it is reasonable that at least, microorganism species in Table 2 above which are other than Mycobacteroides abscessus subsp. abscessus, that is, Priestia sp., Bacillus sp., Neobacillus sp., Fredinandcohnia sp., Metabacillus sp., Litchfieldia sp., Robertmurraya sp., Mycobacteroides sp., Alkalihalobacillus sp., Brevibacillus sp., Paenibacillus sp., and Ammoniphilus sp. are handled as species closely related to Bacillus sp..

Embodiment Example 4: Making a plasmid for BI gene deletion, and a BI gene-deleted strain, as well as analyzing inulin generated

In the present Embodiment Example 4, a procedure for making a plasmid for SD33 strain BI gene deletion and a deletion strain were described as a method of carrying out planned breeding improvement of inulin synthesizing bacteria SD33 strain isolated from nature world into a strain suitable for synthesizing a linear inulin. Additionally, it has been made evident that by deleting the present gene, synthesis of a linear inulin where a culture supernatant is used is made feasible.

Firstly, an overview of a plasmid for BI gene deletion which is used in the present method is stated below. The plasmid for BI gene deletion is a plasmid by subcloning a DNA fragment for BI gene deletion on a plasmid having an optional drug resistance gene. The DNA fragment for BI gene deletion is a fragment where a fragment (A) of approximately 0.5 kb and upstream adjacent to a BI gene is coupled with a fragment (B) of approximately 0.5 kb and downstream adjacent thereto likewise. Firstly, by means of a first-time PCR, an upstream fragment (A) of the BI-like gene and a downstream fragment (B) thereof are prepared, and on this occasion, a primer designed so that a sequence of 10~30 bases on an upstream side of the downstream fragment (B) is added to a downstream terminus of the upstream fragment (A) is used. Primers used in the present Embodiment Example 4 are shown in Table 3 below.

[Table 3]

|  | Primer name | Sequence (5'-3') | Sequence number |
|---|---|---|---|
| (1) | Sma1-IS s | AAAAACCCGGGAACCCATTCCGTTCCTGCAGG | 5 |
| (2) | Sma1-IS as | CGGTCAAATCATTTCCTTAGCGATTTGCCCCAAAT | 6 |
| (3) | ORF3-Xba1 s | TAAGGAAATGATTTGACCG | 7 |
| (4) | ORF3-Xba1 as | AAAAATCTAGAGATCATGGCGTTTTCCAGGA | 8 |
| (5) | pHY300 UP1 | TCATTAGTTGGCTGGTTA | 9 |
| (6) | BI start as | ATTCATAACGGTCATCCTTCT | 10 |

Followingly, by making PCR fragments (A), (B) prepared at a first time be templates, and carrying out a second-time PCR through using an upstream side primer of an upstream fragment and a downstream side primer of a downstream fragment, an anneal is generated between a downstream terminus of the upstream fragment and an upstream terminus of the downstream fragment, and as a result of the PCR, a DNA fragment where the (A) and the (B) are coupled with each other can be obtained. Then, a plasmid for BI gene deletion can be obtained by inserting a DNA fragment obtained through a second-time PCR, into a plasmid to be used in an optional method. Specifically, an SD33 strain genome DNA was made to be a template, a PCR was carried out through using primers (1) & (2), (3) & (4) shown in Table 3 above, two amplification fragments obtained were made to be a template, and a PCR was carried out through using primers (1) & (4) shown in Table 3 above. A PCR in order to amplify DNA fragments was in accordance with statements of the present Embodiment Example 2. Amplification fragments obtained had double digestion carried out by means of restriction enzymes Sma I and Xba I (Takara Bio), and were subcloned in an Sma I - Xba I region of pHY300PLK (Takara Bio). DNA Ligation Kit, Mighty Mix (Takara Bio) was used for ligation. The plasmid was made to be produced by E. coli DH5α transformants and was prepared in an alkali SDS method. By making the plasmid obtained be a template, and carrying out a sequence analysis through using a primer (5) shown in Table 3 above, it was made certain that a target base sequence was incorporated. The present plasmid was made to be a plasmid for BI gene deletion.

Next, by transforming an SD33 strain through using a plasmid for BI gene deletion, obtained, and by means of two-time crossover homologous recombination, a strain which has its BI gene deleted has been obtained. Specifically, 100 μL of glycerol stock of SD33 strain was inoculated into 5 mL of a TM culture medium stuck onto a 300 mL capacity conical flask, and was shake-cultured for 5 hours on conditions of 30°C, 130 rpm. After microbial bodies were collected by means of a centrifugation of 5000 × g, 2 minutes, and were suspended in 5 mL of 50 mM Tris-HCl buffer (pH7.5) and cleansed, a suspension solution was transferred into a 15 mL capacity tube and was given a centrifugation of 5000 × g, 2 minutes. The microbial bodies were suspended in 5 mL of 50 mM Tris-HCl buffer (pH8.5) and were shaken for 60 minutes on conditions of 37°C, 85 rpm. 500 μL of the suspension solution was collected, and had its microbes gathered by means of a centrifugation of 5000 × g, 1 minute, and had its supernatant removed. After 5 μL of 300 ng/μL solution of a plasmid for BI gene deletion and 50 μL of 70 mM phosphate buffer (pH6.3) containing 0.5M sodium sulfate were mixed with each other, they were added to a tube into which microbial bodies were put, were suspended by means of a vortex mixer, and were statically placed for 5 minutes. 150 μL of 70 mM phosphate buffer (pH6.3) containing PEG by 40% was added to a solution in a tube, and was suspended by means of a vortex mixer. After it had its microbes gathered by means of a centrifugation of 3000 × g, 5 minutes, was suspended in 1 mL of a TM culture medium containing 20 mM $MgCl_2$, and was transferred into a 15 mL capacity tube, it was shaken for 60 minutes on conditions of 37°C, 85 rpm. After microbial bodies were collected by means of a centrifugation of 5000 × g, 1 minute, they were inoculated into an LB culture medium containing 10 μg/mL tetracycline and were cultured for 3 days under conditions of 37°C, and a transformant was obtained.

One platinum loop of colonies obtained was inoculated into 3 mL of an LB liquid culture medium containing 10 μg/mL tetracycline, and was shake-cultured for 8 hours on conditions of 30°C, 130 rpm. 50 μL of a culture solution was smeared onto the same agar culture medium, and was cultured for 24 hours under conditions of 35°C. With single colonies obtained being templates, a colony PCR was carried out by using a primer (5) being a universal primer of pHY300PLK and a primer (6) designed on a basis of a sequence near a transcription initiation site of a BI gene, which are listed in Table 3 above.

A colony PCR was carried out by using EmeraldAmp MAX PCR Master Mix (Takara Bio). A colony PCR reaction solution was prepared by adding 1 μL of a microbial body suspension solution diluted as appropriate, 2 pmol respectively of sense- and antisense primers, and 5 μL of EmeraldAmp MAX PCR Master Mix (2×Premix), and further, making a total amount of reaction solution be 10 μL. Conditions for PCR reactions were made to be one cycle of 30 seconds under conditions of 98°C, and 30 cycles of three-step temperature changes of 10 seconds under conditions of 98°C, 30 seconds under conditions of 55°C, and 1~5 minutes (adjusted in accordance with target amplification products, guidelines of 1 minute per 1 kb) under conditions of 72°C. Additionally, sequence reactions were carried out by using a BigDye Terminator v3.1 Cycle Sequencing Kit (Thermo Fisher Scientific), and sequences were analyzed by using an Applied Biosystems 3730x1 DNA Analyzer (Thermo Fisher Scientific). Compositions of culture media used are stated below. For reagents which has nothing explained in particular, commercially available special-grade products were used.

Culture media used

LB culture medium: 10 g/L of tryptone, 5 g/L of yeast extract, 5 g/L of NaCl, (15 g/L of agar powder)
Basal culture medium: 10 g/L of sucrose, 5 g/L of soybean peptone, 1 g/L of yeast extract, 0.5 g/L of $KH_2PO_4$, (15 g/L of agar powder), adjusted to pH7.2 with NaOH
TM culture medium: 10 g/L of glucose, 10 g/L of soybean peptone, 5 g/L of bonito meat extract, 2 g/L of yeast extract, 10 mg/L of $FeSO_4 \cdot 7H_2O$, 10 mg/L of $MnSO_4 \cdot 7H_2O$, 1 mg/L of $ZnSO_4 \cdot 7H_2O$, adjusted to pH7.0 with NaOH

By means of a colony PCR above stated, a colony where amplification fragments of approximately 500 bp were obtained was made to be one-time crossover recombinant strain. After a colony of the one-time crossover recombinant strain was inoculated into 3 mL of a basal culture medium and was shake-cultured for 8~12 hours on conditions of 30°C, 130 rpm, that in an amount of 1/100 was inoculated into 3 mL of the same culture medium. After this is repeatedly done 4 times, a culture solution was smeared onto the same agar culture medium and was cultured for one night under conditions of 35°C. After an agar plate after culturing was applied in a pressed fashion to a velvet cloth fixed on a foundation and sterilized by means of an autoclave to attach a colony thereto, an LB agar culture medium containing no antibiotics and an LB agar culture medium containing 10 μg/mL tetracycline were applied in a pressed fashion one by one, and the colony was transcribed and was cultured for one night under conditions of 35°C. In the present test, a colony which grew on an LB agar culture medium containing no antibiotics and did not grow on an LB agar culture medium containing 10 μg/mL tetracycline was selected, and with this being a template, a colony PCR was carried out by using primers (1) and (4), and a strain where amplification fragments were shorter than those in an SD33 original strain, and where base sizes around 1000 bp were obtained was made to be a BI gene-deleted strain (hereinafter, SD33_ΔBI strain).

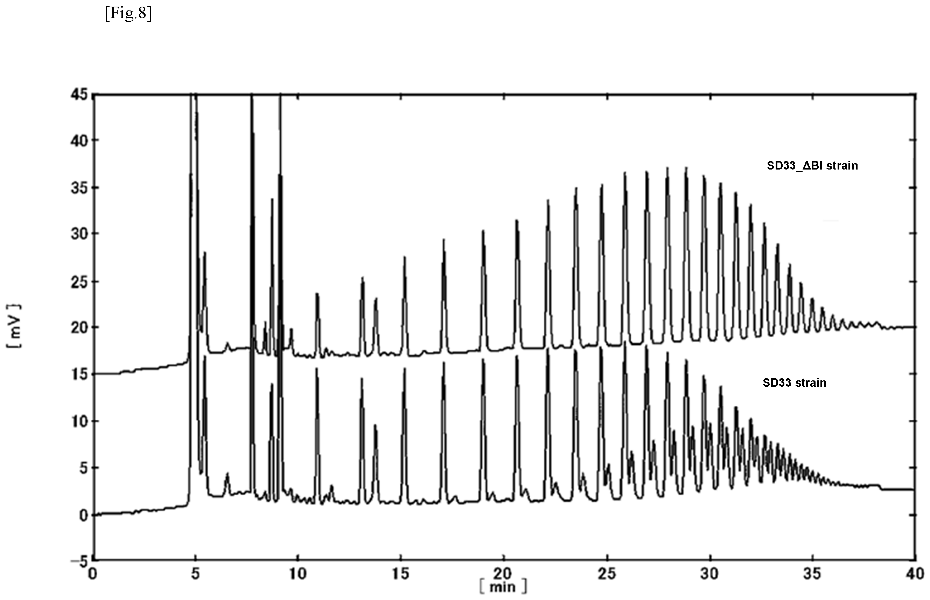

Next, one platinum loop of an SD33 strain and that of an SD33_ΔBI strain were inoculated into 3 mL of a basal culture medium, and were shake-cultured for 24 hours under conditions of 30°C, 130 rpm. Supernatants were collected by means of a centrifugation of 12000 × g, 5 minutes, and after respective culture supernatants had sucrose added so as to be Bx40, and were retained for 24 hours under conditions of 37°C, they had their enzymes deactivated by being retained for 5 minutes under conditions of 100°C, and saccharide compositions of reaction solutions were analyzed by means of HPAEC-PAD. Peaks based on respective HPAEC-PAD analyses for reaction products obtained by making respective culture supernatants of an SD33 strain and an SD33_ΔBI strain react with Bx40 sucrose are shown in Fig.8.

As shown in Fig.8, in a reaction solution where a culture supernatant of an SD33 strain was used, a branching inulin was recognized besides a linear inulin. Whereas, in a reaction solution where a culture supernatant of an SD33_ΔBI strain was used, a linear inulin was exclusively recognized, and at elution times of 20~30 minutes, peak area other than that of a linear inulin was less than 1% of total peak area.

EP 4 759 935 A1

Embodiment Example 5: Analyzing a sucrose reaction products of E. coli recombinant enzymes of BI and IS of various homology

In the present Embodiment Example 5, it has been made evident that even a protein which is considerably low, 67.9%, in homology with a causal protein (BI) clarified in Embodiment Example 3 will be a cause of generating branching inulin, and as examples of inulin synthesizing bacteria holding a gene encoding the causal protein, Priestia sp., Bacillus sp., Neobacillus sp., Fredinandcohnia sp., Metabacillus sp., Litchfieldia sp., Robertmurraya sp., Mycobacteroides sp., Alkalihalobacillus sp., Brevibacillus sp., Paenibacillus sp., and Ammoniphilus sp. were specified.

Out of base sequences encoding various BI-like proteins represented in bold letters on Table 2 above, base sequences encoding amino acid sequences from which sequences of signal peptides predicted by means of SignalP-5.0 and those of termination codons had been excluded were artificially synthesized (sequence numbers 38~47, entrusted to Integrated DNA technologies). Unknown bases at two locations in information of base sequences encoding BI-like proteins derived from Bacillus sp. LL01 strains were presumed from base sequences of related BI-like proteins and were considered to be guanine bases, and genes were artificially synthesized. PCRs were carried out by making respective base sequences artificially synthesized be templates and using primer pairs corresponding, and as in Embodiment example 2, E. coli recombinant enzyme expression systems were built up, and recombinant enzymes were purified. A PCR in order to amplify DNA fragments was in accordance with statements of the present Embodiment Example 2. As BI-like proteins (ATN45517.1) derived from Salipaludibacillus agaradhaerens WDG185 strain were not normally expressed in E. coli, they have been excluded from tests thereafter. A list of primers used in Embodiment example 5 is shown in Table 4 below.

[Table 4]

| Primer name | Sequence (5'-3') | Sequence number |
|---|---|---|
| F. onubensis_BI_s | GGAGATATACATATGGCAGAAACAGAGGTAACG | 11 |
| F. onubensis_BI_as | GTGGTGGTGGTGGTGTTGCTTAATCCAACCAGG | 12 |
| M. abscessus_BI_s | GGAGATATACATATGGATGAAACCGTAACGACC | 13 |
| M. abscessus_BI_as | GTGGTGGTGGTGGTGTTGTTTGATCCATCCCTG | 14 |
| A. krulwichiae_BI_s | GGAGATATACATATGGAGGTCGCGGACACGACT | 15 |
| A. krulwichiae_BI_as | GTGGTGGTGGTGGTGCTTAATCCAACCAGGGCT | 16 |
| R. korlensis_BI_s | GGAGATATACATATGGATGAAACCGTAACGACA | 17 |
| R. korlensis_BI_as | GTGGTGGTGGTGGTGTTGTTTGATCCAACCTTG | 18 |
| B. brevis_BI (B. formosus_BI)_s | GGAGATATACATATGGAGGGCGGTGAGCAAACG | 19 |
| B. brevis_BI (B. formosus_BI)_as | GTGGTGGTGGTGGTGTTGCTTGATCCAACCTGG | 20 |
| P. elgii_BI_s | GGAGATATACATATGCAGGGGGGAGAACAGACA | 21 |
| P. elgii_BI_as | GTGGTGGTGGTGGTGCTTGATCCAACCCTGCGG | 22 |
| LL01_BI_s | GGAGATATACATATGGAGGTCACAGACACGACT | 23 |
| LL01_BI_as | GTGGTGGTGGTGGTGTTTGATCCATCCCTGTGG | 24 |
| S. agaradhaerens_BI_s | GGAGATATACATATGGAGGACACTGATTCACCT | 25 |
| S. agaradhaerens_BI_as | GTGGTGGTGGTGGTGTTTTATCCAACCTTGTGG | 26 |
| OV191_BI_s | GGAGATATACATATGGAAAAAAAAAATCCGCAA | 27 |
| OV191_BI_as | GTGGTGGTGGTGGTGATTGATCGAGCCTGGAGC | 28 |
| OV191_IS_s | GGAGATATACATATGAAAGCGCCGACTACGTCT | 29 |
| OV191_IS_as | GTGGTGGTGGTGGTGGCCGAAATCGCGGATTTC | 30 |
| LL01_IS_s | GGAGATATACATATGAGCTCTAACTGGAATATT | 31 |
| LL01_IS_as | GTGGTGGTGGTGGTGCCTTAAAGATCTACCGTA | 32 |

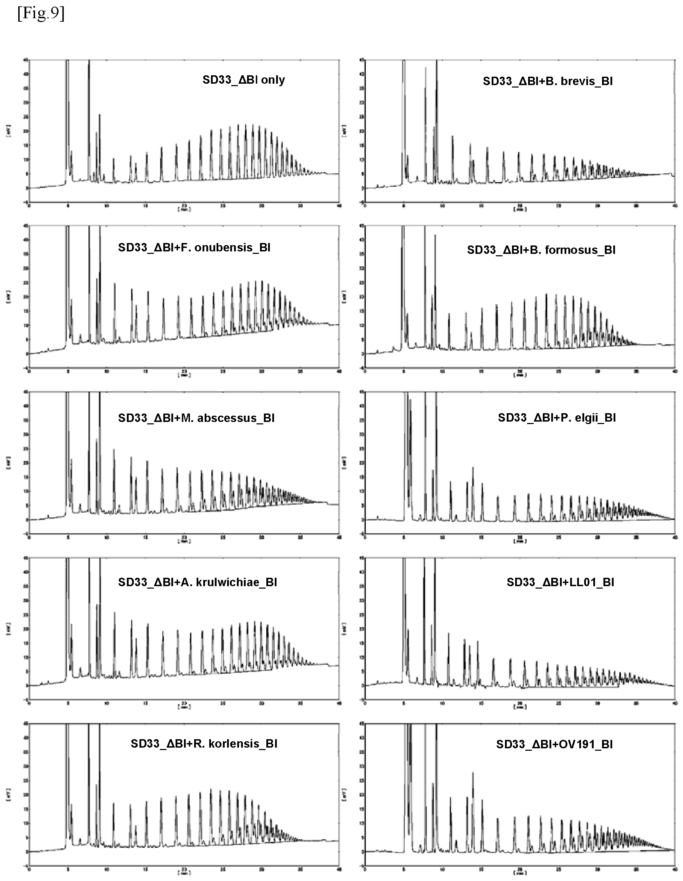

Next, after each of the purified BI-like recombinant enzymes made and a culture supernatant (containing IS enzyme) of an SD33_ΔBI strain were respectively added to Bx40 sucrose solution so as to be 2U/g sucrose, and were retained for 24 hours under conditions of 37°C, they had their enzymes deactivated by being retained for 5 minutes under conditions of 100°C, and saccharide compositions of reaction solutions were analyzed by means of HPAEC-PAD. Peaks based on respective HPAEC-PAD analyses for reaction products obtained by mixing a culture supernatant of an SD33_ΔBI strain with various purified BI-like recombinant enzymes to make them react with Bx40 sucrose are shown in Fig.9. Calculation of enzyme activity was in accordance with statements of the present Embodiment Example 1.

As shown in Fig.9, alone with a culture supernatant of an SD33_ΔBI strain, an inulin to be generated was linear. Additionally, branching inulin ratios (sum of peak area of branching inulin/ total peak area × 100) at elution times of 20~30 minutes (polymerization degree 9~16), on an occasion of mixing each of the purified BI-like recombinant enzymes with a culture supernatant of an SD33_ΔBI strain and making them react with Bx40 sucrose are shown in Table 5 below.

[Table 5]

|  | Branching inulin ratio (%) |
|---|---|
| SD33_ΔBI only | 0.3 |
| SD33_ΔBI+F. onubensis_BI | 12.1 |
| SD33_ΔBI+M. abscessus_BI | 33.1 |
| SD33_ΔBI+A. krulwichiae_BI | 13.3 |
| SD33_ΔBI+R. korlensis_BI | 21.1 |
| SD33_ΔBI+B. brevis_BI | 38.9 |
| SD33_ΔBI+B. formosus_BI | 15.7 |
| SD33_ΔBI+P. elgii_BI | 26.3 |
| SD33_ΔBI+LL01_BI | 34.3 |
| SD33_ΔBI+OV191_BI | 32.7 |

As shown in Table 5 above, a branching inulin ratio at elution times of 20~30 minutes (polymerization degree 9~16) was 0.3%. Meanwhile, as shown in Fig.9, branching inulin peaks came to be detected between linear inulin peaks at elution times of 20 minutes or later because any BI-like recombinant enzyme by 67.9% or higher in homology with sequence number 36 had been added, and a count thereof was 10 or more. Additionally, as shown in Table 5 above, said branching inulin ratio at elution times of 20~30 minutes (polymerization degree 9~16) was above 10% in a case where any BI was used. In a case where each of the BI-like recombinant enzymes was alone made to react with Bx40 sucrose, products of saccharide transfer, which were 3~5 in polymerization degree, were generated in any case, as in a case of an SD33 strain BI.

Embodiment Example 6: Deliberating relatedness between homology of an IS gene and an activity of synthesizing linear inulin

In the present Embodiment Example 6, it has been made evident that even a protein which is considerably low, 67.3%, in homology with an IS protein clarified in Embodiment Example 2 has an inulosucrase activity of generating a linear inulin.

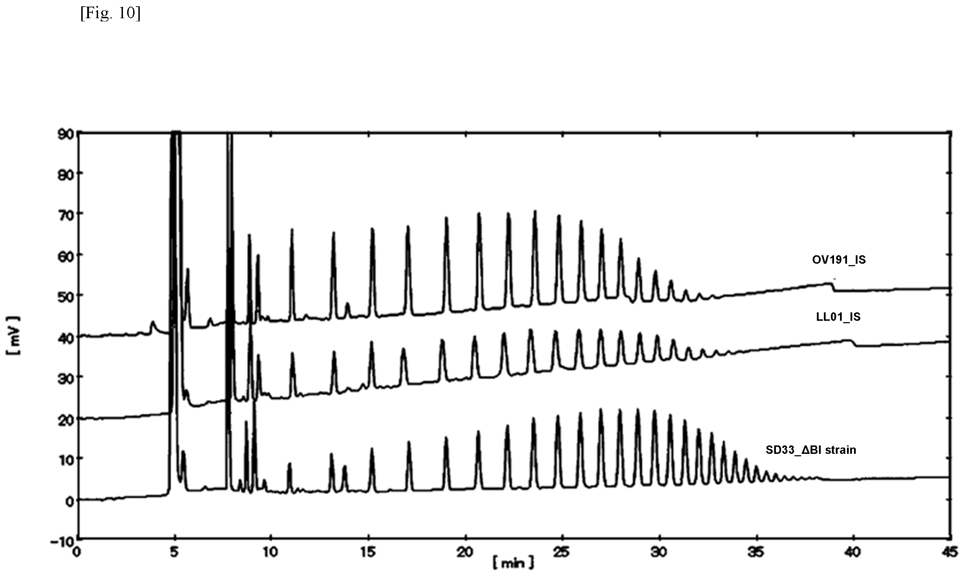

On a basis of base sequences encoding an IS-like protein of Paenibacillus sp. OV191 strain which was 67.3% in homology with an amino acid sequence (sequence number 34) of IS of an SD33 strain, and an IS-like protein of Bacillus sp. LL01 strain which was 68.8% (hereinafter, referred to as 'OV191_IS' and 'LL01_IS', respectively), base sequences encoding amino acid sequences from which sequences of signal peptides predicted by means of SignalP-5.0 and those of termination codons had been excluded were artificially synthesized (sequence numbers 48, 49), and E. coli recombinant enzyme expression systems were built up, and recombinant enzymes were purified, as in Embodiment Example 5. Peaks based on respective HPAEC-PAD analyses for reaction products obtained by making a culture supernatant of an SD33_ΔBI strain, as well as making OV191_IS and LL01_IS, react with Bx40 sucrose are shown in Fig.10.

As shown in Fig.10, an inulin generated when recombinant enzymes of two making reactions were made to react with Bx40 sucrose solution showed a peak pattern of a linear inulin as in a case where an SD33_ΔBI strain was used although it had few peaks on its high polymerization degree side. From the above, it has turned out that even proteins low, 67.3% and 68.8%, in homology with IS of an SD33 strain have an inulosucrase activity of generating a linear inulin.

Embodiment Example 7: Measuring DFA III yield in a one-pot synthesis

In the present Embodiment Example 7, it has been made evident that even a protein which is considerably low, 67.9%, in homology with a causal protein (BI) clarified in Embodiment Example 3 will be a cause of generating a branching inulin and will be a cause of lowering yield of a DFA III generation from sucrose, which uses a culture supernatant of an SD33 strain BI gene-deleted strain of Embodiment Example 4, and inulin fructotransferase.

Although DFA III is generated when inulin fructotransferase (IFT) is made to act on inulin, yield of DFA III generation becomes lower when an inulin containing a branching inulin is used, than in a case where a linear inulin is used. When even in a case of adding IS and IFT to a sucrose solution and carrying out a one-pot reaction of DFA III, a culture supernatant of a wild strain such as an SD33 strain is used as IS, this culture supernatant contains BI as well, and therefore, yield of DFA III generation becomes lower than in a case of using a purified IS. Firstly, one-pot reactions were compared between a culture supernatant of an SD33 strain and a culture supernatant of an SD33_ΔBI strain.

After a Bx40 sucrose solution which had IFT (made by Nippon Beet Sugar Manufacturing Co., Ltd.) derived from Arthrobacter sp. H65-7 strain added so as to be 4U/g sucrose had any of (1) a culture supernatant (2U/g sucrose) of an SD33 strain or (2) a culture supernatant (1U/g sucrose) of an SD33_ΔBI strain, added, and was retained for 90 hours under conditions of 50°C, it was stopped from reacting enzymatically, by being retained for 10 minutes under conditions of 100°C. Next, it was diluted by 10 times with ultrapure water, in order to analyze a saccharide composition of a reaction solution. After in order to hydrolyze with acid, sucrose yet to react in this reaction solution, 1 mL of 10-times diluted solution had 100 μL of 1N HCl added and was retained for 2 hours under conditions of 60°C, it had 100 μL of 1N NaOH added and had its pH returned, and thereafter, was put in HPLC analyses. HPLC conditions were made to be, Column: Shodex SUGAR KS-801 (Guard column: SUGAR KS-G), Column temperature: 60°C, Solvent: 1/20000N NaOH, Flow rate: 1.0 mL/minutes, Detection: RI, Injection amount: 5μL of 1 w/v% water solution. From a peak area obtained, a saccharide composition % (equivalent to yield of DFA III synthesis with respect to sucrose) of DFA III was calculated by means of a calculation formula below. Yield of DFA III synthesis in a case where respective purified BI-like recombinant enzymes were made to react with sucrose is shown in Table 6 below.

[Calculation formula]

Saccharide composition % of DFA III = [Peak area of disaccharides after degradation] / [Total peak area of saccharides before degradation] $\times$ 100

[Table 6]

|  | DFA III (%) |
| --- | --- |
| SD33 supernatant | 27.0 |
| SD33_ΔBI supernatant | 38.6 |
| +F. onubensis_BI | 30.0 |
| +M. abscessus_BI | 26.5 |
| +A. krulwichiae_BI | 30.3 |
| +R. korlensis_BI | 29.8 |
| +B. brevis_BI | 27.2 |
| +B. formosus_BI | 26.9 |
| +P. elgii_BI | 27.2 |
| +OV191_BI | 26.8 |
| +LL01_BI | 26.9 |

Yield of DFA III synthesis in a case where a culture supernatant of an SD33 strain listed in Table 6 above was used was 27.0%, whereas that in a case where a culture supernatant of an SD33_ΔBI strain was used improved greatly to 38.6%.

Next, in order to clarify homology of BI which lowers reaction yield of a one-pot reaction of DFA III, a following experiment was carried out by using BI-like recombinant enzymes made in Embodiment Example 5. Yield of DFA III synthesis was measured by adding, to a Bx40 sucrose solution which had IFT (made by Nippon Beet Sugar Manufacturing Co., Ltd.) added so as to be 4U/g sucrose, a mixture solution (1U/g sucrose respectively) of a culture supernatant of an SD33_ΔBI strain of 4U/g sucrose and a purified BI-like recombinant enzyme, to make it react enzymatically under reaction conditions identical to the above. Yield of DFA III synthesis with respect to sucrose has turned out to be as shown in Table 6 above. Since even if any of the BI-like recombinant enzymes was mixed, yield of DFA III became lower than in a case of a culture supernatant of an SD33_ΔBI strain being exclusively present, it was able to be made certain that a BI-like protein which is by 67.9% or higher in homology with BI of an SD33 strain has, in any case, an enzyme activity equivalent to that of BI of an SD33 strain and lowers yield of DFA III in a one-pot DFA III synthesis reaction.

Lastly, it was deliberated whether even in a case of combining BI and IS which were derived from other bacterial strains, reaction yield of a one-pot reaction of DFA III would be lowered. After by adding IS (1U/g sucrose) derived from Paenibacillus sp. OV191 strain or adding both IS and BI (1U/g sucrose, respectively) therefrom to a Bx40 sucrose solution which had an IFT (4U/g sucrose) added, and retaining them for 90 hours under conditions of 50°C, they were made to react, they were, by being retained for 10 minutes under conditions of 100°C, stopped from reacting enzymatically, and yield of DFA III synthesis with respect to sucrose was measured. Additionally, combined IS and BI which were derived from Bacillus sp. LL01 strain were also made to react enzymatically likewise, and yield of DFA III synthesis was measured. Yield of DFA III synthesis in a case where IS and BI recombinant enzymes which were derived from Paenibacillus sp. OV191 strain and Bacillus sp. LL01 strain were respectively made to react with sucrose is shown in Table 7 below.

[Table 7]

|  | DFA III (%) |
|---|---|
| OV191_IS | 30.1 |
| OV191_IS+BI | 25.0 |
| LL01_IS | 33.6 |
| LL01_IS+BI | 29.2 |

Yield of DFA III synthesis has turned out to be as shown in Table 7. From the above, it was able to be made certain that even in a case where IS and BI which are derived from Paenibacillus sp. OV191 strain and Bacillus sp. LL01 strain are used, DFA III yield is lowered as in an SD33 strain.

| 0-1 | **Form PCT/RO/134** **This identification of the deposited microorganism or other biological material (PCT Rule 13bis} was made by the right.** |  |
|---|---|---|
| 0-1-1 | | **JP0-PAS i480** |
| 0-2 | **International application No.** | |
| 0-3 | **Applicant's or agent's file reference** | **PCT0074** |

| 1 | **The** Indications **made below relate to the deposited microorganism or other biological material referred to In the description.** | |
|---|---|---|
| 1-1 | **Paragraph number** | **0042** |
| 1-3 | **Identification** of **deposit** | **NITE Patent Microorganisms Depositary** |
| 1-3-1 | **Name of depositary institution** | **National Institute of Technology and Evaluation** |
| 1-3-2 | **Address** of **depositary institution** | **#122, 2-5-8 Kazusakamatari, Kisarazu-shi, Chiba 292-0818, Japan** |
| 1-3-3 | **Date of deposit** | **May 22, 2023** |
| 1-3-4 | **Accession Number** | **NPMD NITE BP-03890** |
| 1-5 | **Designated states for which indications are made** | **All designated states** |

(continued)

| For receiving Office use only | | |
|---|---|---|
| 0-4 | This sheet was received with the international application (Yes / No) | |
| 0-4-1 | Authorized officer | |
| For International Bureau use only | | |
| 0-5 | This sheet was received by the international Bureau on: | |
| 0.5.1 | Authorized officer | |

[Sequence Listing]

P0074US_ST26.xml

**Claims**

1. Linear inulin synthesizing bacteria such as Priestia sp., Bacillus sp., Neobacillus sp., Fredinandcohnia sp., Metabacillus sp., Litchfieldia sp., Robertmurraya sp., Mycobacteroides sp., Alkalihalobacillus sp., Brevibacillus sp., Paenibacillus sp., and Ammoniphilus sp., as well as linear inulin synthesizing bacteria which are other than those linear inulin synthesizing bacteria and which are one, alternatively, two or more ones selected out of a group made up of linear inulin synthesizing bacteria being species closely related to Bacillus sp., which have a gene represented by at least any of (a)~(e) below deleted or inactivated, which have a gene represented by at least any of (h)~(m);

   (a) a gene which encodes a protein made up of a base sequence having an identity of 70% or higher with a base sequence indicated by sequence number 35,
   (b) a gene which hybridizes under stringent conditions with a complementary strand of a gene made up of a base sequence indicated by sequence number 35,
   (c) a gene which encodes a protein made up of an amino acid sequence indicated by sequence number 36,
   (d) a gene which encodes a protein made up of an amino acid sequence having 1~10 amino acid residues deleted, substituted, added, or inserted in an amino acid sequence indicated by sequence number 36,
   (e) a gene which encodes a protein made up of an amino acid sequence having an identity of 67.9% or higher with an amino acid sequence indicated by sequence number 36,
   (h) a gene which is of a base sequence indicated by sequence number 33,
   (i) a gene which is made up of a base sequence having an identity of 70% or higher with a base sequence indicated by sequence number 33, as well as encodes a protein having an activity of catalyzing a reaction of making β-2,1 transfer of a fructofuranosyl group of a sucrose to another sucrose or a terminus fructose portion of a product of that saccharide transfer,
   (j) a gene which hybridizes under stringent conditions with a complementary strand of a gene made up of a base sequence indicated by sequence number 33, as well as encodes a protein having an activity of catalyzing a reaction of making β-2,1 transfer of a fructofuranosyl group of a sucrose to another sucrose or a terminus fructose portion of a product of that saccharide transfer,
   (k) a gene which encodes a protein that is made up of an amino acid sequence indicated by sequence number 34, and is to be microbe-extracellularly secreted,
   (l) a gene which encodes a protein that is made up of an amino acid sequence having 1~10 amino acid residues deleted, substituted, added, or inserted in an amino acid sequence indicated by sequence number 34 as well as has an activity of catalyzing a reaction of making β-2,1 transfer of a fructofuranosyl group of a sucrose to another sucrose or a terminus fructose portion of a product of that saccharide transfer, and is to be microbe-extracellularly secreted,
   (m) a gene which encodes a protein that is made up of an amino acid sequence having an identity of 67.3% or higher with an amino acid sequence indicated by sequence number 34 as well as has an activity of catalyzing a reaction of making β-2,1 transfer of a fructofuranosyl group of a sucrose to another sucrose or a terminus fructose portion of a product of that saccharide transfer, and is to be microbe-extracellularly secreted.

2. Linear inulin synthesizing bacteria according to (1), wherein linear inulin synthesizing bacteria of said inulin synthesizing bacteria have a feature shown in (f) or (g) below;

(f) Linear inulin synthesizing bacteria which generate an inulin which has its bonds between fructose residues be made up of β-2,1 bonds exclusively, by inoculating one platinum loop of microbial body into 3 mL of a culture medium whose composition is as below, shake-culturing it on conditions of 30°C, 24 hours, 130 rpm, and thereafter, making a culture supernatant thereof have sucrose added so as to be Bx40, and making it react at 37°C for 24 hours, <Culture medium composition>

10 g/L of sucrose, 5 g/L of soybean peptone, 1 g/L of yeast extract, 0.5 g/L of $KH_2PO_4$, adjusted to pH 7.2 with NaOH,

(g) Linear inulin synthesizing bacteria wherein when a reaction product obtained by inoculating one platinum loop of microbial body into 3 mL of a culture medium whose composition is as below (I), shake-culturing it on conditions of 30°C, 24 hours, 130 rpm, and thereafter, making a culture supernatant thereof have sucrose added so as to be Bx40, and making it react at 37°C for 24 hours is put in HPAEC-PAD analyses on conditions below (II), peaks of elution times identical to those of an inulin made up of β-2,1 bonds exclusively are seen at elution times of 20~30 minutes (polymerization degrees 9~16), while area of peaks other than those are less than 1% of total peak areas,

<(I) Culture medium composition>
10 g/L of sucrose, 5 g/L of soybean peptone, 1 g/L of yeast extract, 0.5 g/L of $KH_2PO_4$, adjusted to pH 7.2 with NaOH,
<(II) Conditions of HPAEC-PAD analyses>
Column, CarboPac PA1 4 × 250 mm; Guard column, CarboPac PA1 4 × 50 mm; Injection amount, 5 μL of 0.1% water solution of a sample; Flow rate, 1.0 mL/minute; Elution, NaOH 100 mM constant, AcONa gradient (0~60 minutes, 0 → 600 mM); Detection, pulsed amperometry.

3. Linear inulin synthesizing bacteria according to claim 1, wherein a gene made up of a base sequence which is of said sequence number 35 or which has an identity of 75% or higher with the sequence number has features shown below;

when a protein made up of an amino acid sequence encoded by a gene made up of a base sequence which is of said sequence number 35 or which has an identity of 75% or higher with the sequence number, and a protein made up of an amino acid sequence indicated by sequence number 34 are respectively added to a Bx40 sucrose solution so as to be 2U/g sucrose and react at 37°C for 24 hours, and when a reaction product obtained is put in HPAEC-PAD analyses on conditions below, ten or more peaks of elution times identical to those of a linear inulin which is obtained by a reaction of a protein made up of an amino acid sequence indicated by sequence number 34 with a sucrose solution of Bx40, and which has its bonds between fructose residues be made up of β-2,1 bonds exclusively are obtained at elution times of 20 minutes or later (polymerization degree 9 or more), while ten or more peaks which appear between peaks of said linear inulin and are of a branching inulin having a structure where in a way of bonding other than β-2,1 bonds, a fructofuranosyl group is bonded to any fructose portion of a linear inulin are detected, and a branching inulin ratio (sum of peak area of branching inulin/ total peak area × 100) reaches 10% or higher at elution times of 20~30 minutes (polymerization degree 9~16),
<Conditions of HPAEC-PAD analyses>
Column, CarboPac PA1 4 × 250 mm; Guard column, CarboPac PA1 4 × 50 mm; Injection amount, 5 μL of 0.1% water solution of a sample; Flow rate, 1.0 mL/minute; Elution, NaOH 100 mM constant, AcONa gradient (0~60 minutes, 0 → 600 mM); Detection, pulsed amperometry.

4. A purified enzyme, crudely-made enzyme, or enzyme-containing matter, culture solution, culture supernatant, or culture microbial body obtained from linear inulin synthesizing bacteria according to any one claim of claim 1 to claim 3, or a treatment product thereof.

5. A method for producing a linear inulin, which has a process of culturing linear inulin synthesizing bacteria according to any one claim of claim 1 to claim 3.

6. A method for producing a linear inulin, which has a process of obtaining a reaction product by making at least one of a purified enzyme, crudely-made enzyme, or enzyme-containing matter, culture solution, culture supernatant, or culture microbial body obtained from linear inulin synthesizing bacteria according to any one claim of claim 1 to claim 3, or a treatment product thereof react with sucrose.

7. A method for producing a linear inulin according to claim 6, wherein in a case of putting said reaction product in HPAEC-PAD analyses on conditions below, a ratio (sum of peak area of branching inulin/ total peak area × 100) of said branching inulin having a structure where in a way of bonding other than β-2,1 bonds, a fructofuranosyl group is bonded to any fructose portion of a linear inulin which has its bonds between fructose residues be made up of β-2,1 bonds is less than 1% at elution times of 20~30 minutes (polymerization degree 9~16),
<Conditions of HPAEC-PAD analyses>
Column, CarboPac PA1 4 × 250 mm; Guard column, CarboPac PA1 4 × 50 mm; Injection amount, 5 μL of 0.1% water solution of a sample; Flow rate, 1.0 mL/minute; Elution, NaOH 100 mM constant, AcONa gradient (0~60 minutes, 0 → 600 mM); Detection, pulsed amperometry.

8. A method for producing a DFA III, which makes at least one of a purified enzyme, crudely-made enzyme, or enzyme-containing matter, culture solution, culture supernatant, or culture microbial body obtained from linear inulin synthesizing bacteria according to any one claim of claim 1 to claim 3, or a treatment product thereof react with sucrose.

9. A method for producing a DFA III according to claim 8, which uses inulin fructotransferase.

[Fig.1]

EP 4 759 935 A1

[Fig.2]

[Fig. 3]

EP 4 759 935 A1

[Fig.4]

chemical shifts (ppm)

[Fig.5]

[Fig. 6]

EP 4 759 935 A1

[Fig.7]

[Fig.8]

EP 4 759 935 A1

[Fig.9]

[Fig. 10]

**EP 4 759 935 A1**

<table>
<tr><td colspan="2">INTERNATIONAL SEARCH REPORT</td><td>International application No.<br><br>**PCT/JP2023/029646**</td></tr>
</table>

| | |
|---|---|
| **A.** **CLASSIFICATION OF SUBJECT MATTER** | |

*C12P 19/04*(2006.01)i; *C12P 19/12*(2006.01)i; *C12N 1/20*(2006.01)i; *C12N 1/21*(2006.01)i; *C12N 15/31*(2006.01)i; *C12N 15/54*(2006.01)i

FI: C12N1/21; C12N1/20 A; C12N15/31 ZNA; C12N15/54; C12P19/04 C; C12P19/12

According to International Patent Classification (IPC) or to both national classification and IPC

| | |
|---|---|
| **B.** **FIELDS SEARCHED** | |

Minimum documentation searched (classification system followed by classification symbols)

C12P19/04; C12P19/12; C12N1/20; C12N1/21; C12N15/31; C12N15/54

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN); GenBank/EMBL/DDBJ/GeneSeq; UniProt/GeneSeq

| | |
|---|---|
| **C.** **DOCUMENTS CONSIDERED TO BE RELEVANT** | |

| Category* | Citation of document, with indication, where appropriate, of the relevant passages | Relevant to claim No. |
|---|---|---|
| X | JP 2021-177712 A (NIPPON ORIGO KK) 18 November 2021 (2021-11-18)<br>    claims, examples | 4-7 |
| Y | claims, examples | 8, 9 |
| A | claims, examples | 1-3 |
| X | JP 2017-536131 A (DANISCO US INC.) 07 December 2017 (2017-12-07)<br>    claims, examples | 4-7 |
| Y | claims, examples | 8, 9 |
| A | claims, examples | 1-3 |
| Y | JP 2004-305125 A (NIPPON BEET SUGAR MFG CO., LTD.) 04 November 2004<br>(2004-11-04)<br>    paragraph [0015] | 8, 9 |
| A | paragraph [0015] | 1-7 |

✓ Further documents are listed in the continuation of Box C.      ✓ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **05 October 2023** | **17 October 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

36

## INTERNATIONAL SEARCH REPORT

International application No.

**PCT/JP2023/029646**

### C. DOCUMENTS CONSIDERED TO BE RELEVANT

| Category* | Citation of document, with indication, where appropriate, of the relevant passages | Relevant to claim No. |
|---|---|---|
| Y | JP 2005-170855 A (NIPPON BEET SUGAR MFG CO., LTD.) 30 June 2005 (2005-06-30) paragraphs [0017]-[0018] | 8, 9 |
| A | paragraphs [0017]-[0018] | 1-7 |
| Y | JP 2010-233576 A (NIPPON BEET SUGAR MFG CO., LTD.) 21 October 2010 (2010-10-21) example 2 | 8, 9 |
| A | example 2 | 1-7 |
| A | WADA, T. et al. Physicochemical characterization and biological effects of inulin enzymatically synthesized from sucrose. J. Agric. Food Chem. 2005, vol. 53, no. 4, pp. 1246-1253<br>Abstract | 1-9 |
| A | NI, D. et al. Inulin and its enzymatic production by inulosucrase: Characteristics, structural features, molecular modifications and applications. Biotechnol. Adv. 2019, vol. 37, no. 2, pp. 306-318<br>Abstract | 1-9 |
| A | JP 2009-50281 A (FUJI NIHON SEITO KK) 12 March 2009 (2009-03-12) examples | 1-9 |
| A | WO 2022/096864 A1 (INULOX LTD.) 12 May 2022 (2022-05-12) examples | 1-9 |

Form PCT/ISA/210 (second sheet) (January 2015)

## INTERNATIONAL SEARCH REPORT

| International application No. |
| --- |
| **PCT/JP2023/029646** |

**Box No. I          Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  [✓] forming part of the international application as filed:

        [✓] in the form of an Annex C/ST.25 text file.

        [ ] on paper or in the form of an image file.

    b.  [ ] furnished together with the international application under PCT Rule 13*ter*.1(a) for the purposes of international search only in the form of an Annex C/ST.25 text file.

    c.  [ ] furnished subsequent to the international filing date for the purposes of international search only:

        [ ] in the form of an Annex C/ST.25 text file (Rule 13*ter*.1(a)).

        [ ] on paper or in the form of an image file (Rule 13*ter*.1(b) and Administrative Instructions, Section 713).

2.  [ ]  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

    "In the form of an Annex C/ST.25 text file" above should be understood as "in ST. 26 format".

Form PCT/ISA/210 (continuation of first sheet) (January 2015)

## INTERNATIONAL SEARCH REPORT
### Information on patent family members

International application No.

**PCT/JP2023/029646**

| Patent document cited in search report | | | Publication date (day/month/year) | Patent family member(s) | | | Publication date (day/month/year) |
|---|---|---|---|---|---|---|---|
| JP | 2021-177712 | A | 18 November 2021 | (Family: none) | | | |
| JP | 2017-536131 | A | 07 December 2017 | US | 2017/0267981 | A1 | |
| | | | | claims, examples | | | |
| | | | | US | 2022/0002685 | A1 | |
| | | | | claims, examples | | | |
| | | | | WO | 2016/090192 | A1 | |
| | | | | claims, examples | | | |
| | | | | EP | 3253868 | A1 | |
| | | | | claims, examples | | | |
| | | | | CN | 107278226 | A | |
| | | | | claims, examples | | | |
| JP | 2004-305125 | A | 04 November 2004 | (Family: none) | | | |
| JP | 2005-170855 | A | 30 June 2005 | (Family: none) | | | |
| JP | 2010-233576 | A | 21 October 2010 | US | 2006/0051845 | A1 | |
| | | | | example 2 | | | |
| | | | | WO | 2004/078989 | A1 | |
| | | | | example 2 | | | |
| | | | | EP | 1612274 | A1 | |
| | | | | example 2 | | | |
| | | | | EP | 2450452 | A1 | |
| | | | | example 2 | | | |
| | | | | KR | 10-2005-0105933 | A | |
| | | | | example 2 | | | |
| | | | | KR | 10-2012-0003976 | A | |
| | | | | example 2 | | | |
| JP | 2009-50281 | A | 12 March 2009 | JP | 4307259 | B2 | |
| | | | | examples | | | |
| | | | | US | 2004/0241810 | A1 | |
| | | | | examples | | | |
| | | | | WO | 2003/027304 | A1 | |
| | | | | examples | | | |
| | | | | EP | 1457567 | A1 | |
| | | | | examples | | | |
| | | | | CN | 1610750 | A | |
| | | | | examples | | | |
| WO | 2022/096864 | A1 | 12 May 2022 | CN | 116456841 | A | |
| | | | | examples | | | |
| | | | | CA | 3196047 | A1 | |
| | | | | examples | | | |
| | | | | EP | 4240179 | A1 | |
| | | | | examples | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Biotechnology Advances*, 2019, vol. 37, 306-318
- *J. Agric. Food Chem.*, 2005, vol. 53, 1246-1253
- *Nature Protocols*, 2012, vol. 7, 1590-1607